# EUROPEAN PATENT APPLICATION

(11) **EP 0 568 384 A1**
(43) Date of publication of application: **03.11.1993**
(21) Application number: 93303401.9
(22) Date of filing: 30.04.1993
(51) Int. Cl.: C12N 15/12, C07K 13/00, C12N 1/21, G01N 33/68

(54) **Human glutamate receptor proteins and associated DNA compounds**

(30) Priority: 01.05.1992 US 879688; 19.05.1992 US 885912
(71) Applicant: ELI LILLY AND COMPANY, Indianapolis, Indiana 46285 (US)
(72) Inventor: Burnett, James Paul, Jr., Indianapolis, Indiana 46250 (US); Mayne, Nancy Gail, Indianapolis, Indiana 46226 (US); Sharp, Robert Leon, Indianapolis, Indiana 46202 (US); Snyder, Yvonne Marie, Indianapolis, Indiana 46256 (US)
(74) Representative: Hudson, Christopher Mark

(57) **Abstract**

The present invention provides human glutamate receptor proteins and related DNA compounds, useful in assays for potential pharmaceuticals and in methods for molecular biology techniques.

## Description

This invention relates to novel human glutamate receptor proteins and to novel nucleic acid compounds that encode the novel proteins.

In the mammalian central nervous system, L-glutamate serves as a major excitatory neurotransmitter. The interaction of glutamate with its membrane-bound receptors is believed to play a role in many important neuronal processes, including, for example, fast synaptic transmission, synaptic plasticity and long-term potentiation. These processes are fundamental to the maintenance of life and normal human abilities such as learning and memory. Monaghan D.T. *et al.,* 8 *Neuron* 267 (1992).

Pharmacological characterization of receptors for L-glutamate has led to their classification into two families based on their biological function: the ionotropic receptors which are directly coupled to cation channels in the cell membrane, and the metabotropic receptors which function through coupling to G-proteins. A number of ionotropic receptors have been further characterized on the basis of the relatively specific agonists by which they can be activated. One major group comprises those receptors activated by N-methyl-D-aspartate (NMDA), which appears to have multiple allosteric modulatory sites. The other two groups consist of those receptors activated by kainate and/or amino-3-hydroxy-5-methyl-4-isoxozole propionate (AMPA). Collingridge G. L. *et al.,* 40 *Pharmacol. Rev.* 143 (1989).

Molecular cloning studies of rodent ionotropic receptors have recently provided some information on the molecular structure of these proteins. The cDNAs for seven different subtypes of the kainate/AMPA group have been characterized. Heinemann S. *et al.,* PCT publication, WO91/06648 (1991); Keinanen K. *et al., 249 Science* 556 (1990); Sakimura K. *et al., 272 FEBS Lett.* 73 (1990); Werner P. *et al., 341 Nature* 742 (1991); and Bettler B. *et al., 8* *Neuron* 257 (1992). Splice variants, referred to as "flip" and "flop", of some of these have been characterized as well. Sommer B. *et al.,* 249 *Science* 1580 (1990). In addition, two members of the NMDA group have been cloned. Moriyoshi, K. *et al.,* 354 *Nature* 31 (1991) and Meguro H. *et al.,* 357 *Nature* 70 (1992). An NMDA-related protein has also been reported. Kumar K. N. *et al.,* 354 *Nature* 70 (1991). These proteins share varying degrees of homology with one another and are, therefore, believed to represent a gene superfamily. Based on analogy with other better characterized ion channel receptors, glutamate ionotropic receptors are expected to exist *in vivo* within the cell membrane as heteromeric multisubunit assemblies of these subunits. Unwin N., 3 *Neuron* 665 (1989).

Moreover, at least two human glutamate receptors have been reported as cloned. Puckett C. *et* *al., 88 Proc. Nat. Acad. Sci.* 7557 (1991) and Sun W. *et* *al., 89 Proc. Nat. Acad. Sci.* 1443 (1992). The glutamate receptor cloned by Puckett *et al.* was named GluHI and was later identified to be the "flip" version of this particular receptor. The Sun W. *et al.* reference refers to the glutamate receptor they cloned as the HBGF1 receptor and explains that HBGR1 is presumed the "flop" version of GluHI. Sun *et al.* also discloses the possible existence of, but does not describe in detail, a partial clone of HBGR2, or human GluR2.

In addition to its role in normal human physiology, interaction of L-glutamate with its receptors is believed to play a key role in many neurological disorders such as stroke, epilepsy, and head trauma, as well as neurodegenerative processes such as Alzheimer's disease. Olney R. W., 17 *Drug Dev.* *Res.,* 299 (1989). For this reason, understanding the molecular structure of human L-glutamate receptors will be important for understanding these disease processes as well as for furthering the search for effective therapeutic agents. Up to the present, the search for therapeutic agents which will selectively bind and modulate the function of human glutamate receptors has been hampered by the unavailability of homogeneous sources of receptors to use for screens and tests of potential drug candidate compounds. The brain tissues commonly used by pharmacologists presently are derived from experimental animals (non-human) and furthermore contain mixtures of various types of glutamate receptors. In searching for drugs for human therapy it is desirable to use receptors that are more analogous to those in the intact human brain than are the rodent receptors employed to date.

The discovery of human glutamate receptors, therefore, provides a necessary research tool for the development of selective pharmaceutical agents. According to the present invention there is provided human glutamate receptors and functional equivalents thereof which can be used to search for drugs which modulate these receptors.

The present invention provides human glutamate receptor proteins which comprise the amino acid sequence SEQ ID NO:1, amino acid sequence SEQ ID NO:3, or functional equivalents thereof. In particular, the amino acid compound which is SEQ ID NO:1 or SEQ ID NO:3 is preferred.

The invention also provides nucleic acid compounds which comprise a nucleic acid sequence which encodes the amino acid compounds provided. Particularly, nucleic acid compounds which are DNA are preferred. Most preferred are the DNA compounds SEQ ID NO:2 and SEQ ID NO:4 which encode amino acid compounds SEQ ID NO:1 and SEQ ID NO.3, respectively. However, also preferred are those nucleic acid compounds which are sense mRNA.

Also provided by the present invention are nucleic acid vectors comprising nucleic acids which encode SEQ ID NO:1, SEQ ID NO:3, or functional equivalents thereof. Preferred nucleic acid vectors are those which are DNA. Most preferred are DNA vectors which comprise SEQ ID NO:2 or SEQ ID NO:4. The DNA vectors most preferred are plasmids pRS113 and pRS103.

Restriction fragments of the preferred vectors are also provided. Particularly, the approximately 2.9 kb (kilobase) AlwnI/SalI restriction fragment and the approximately 2.9 EcoRI restriction fragment of pRS113 are provided. Also, the 4.2 kb EcoRI/Kpn1 and the 2.8 kb EcoRI/ClaI restriction fragment of pRS103 are provided.

Moreover, DNA vectors of the present invention preferably comprise a promoter positioned to drive expression of said DNA sequence. A preferred DNA expression vector is one wherein the promoter functions in mammalian cells. A more preferred DNA expression vector is one wherein the promoter functions in COS-7 cells. Most preferred COS-7 DNA expression vectors comprise SEQ ID NO:2 or SEQ ID NO:4.

The present invention also provides probes and primers useful for molecular biology techniques. Compounds which encode SEQ ID NO:1 or a part thereof and which are at least 17 consecutive base pairs in length are provided. Preferably, the 17 base pair or more compound is DNA. Most preferred for this use are the DNA compounds which are SEQ ID NO:5, SEQ ID NO:6 and SEQ ID NO:7. Also, compounds which encode SEQ ID NO:3 or a part thereof and which are at least 17 consecutive base pairs in length are provided. Preferably, the 17 base pair or more compound is DNA. Most preferred for this use are the DNA compounds which are SEQ ID NO:8, SEQ ID NO:9, and SEQ ID NO:10.

Further, this invention provides cells in which the nucleic acid compounds of the invention may be harbored. For example, oocytes which comprise a nucleic acid compound which encodes all or part of SEQ ID NO:1 or SEQ ID NO:3 are provided. Oocytes wherein DNA expresses the HSGluR1 receptor or the HSGluR2 receptor are preferred. Most preferred are oocytes wherein sense mRNA expresses the HSGluR1 receptor or the HSGluR2 receptor.

Moreover, oocytes wherein nucleic acid compounds of the invention express functional HSGluR1 receptor or functional HSGluR2 receptor are provided. For example, oocytes wherein nucleic acid compounds of the invention express functional HSGluR2/HSGluR1 complex are provided. Oocytes wherein nucleic acids of the present invention express functional HSGluR2/HSGluR1 complex and wherein GluR3 receptor is also expressed are provided. Oocytes which comprise functional HSGluR2 and which further comprise GluR1 receptor are part of the present invention. Furthermore, oocytes which comprise functional HSGluR2 and which further comprise GluR3 receptor are also provided. Oocytes which comprise functional HSGluR2 receptor and wherein GluR1 receptor is co-expressed, and wherein GluR3 receptor is additionally expressed are also provided. Also, oocytes which comprise functional HSGluR1 and which further comprise functional GluR2 and GluR3 receptors are also provided. An oocyte wherein DNA expresses functional HSGluR1 and/or HSGluR2 receptors is preferred. Most preferred in an oocyte wherein sense mRNA expresses functional HSGluR1 receptor and/or functionlal HSGluR2 receptor.

Other host cells provided by the present invention include those which are transfected with a nucleic acid compound which encodes SEQ ID NO:1, SEQ ID NO:3, or functional equivalents thereof. Preferred cells include host cells transfected with a DNA vector. Host cells wherein the DNA vector comprises the DNA sequence SEQ ID NO:2 or SEQ ID NO:4 are preferred. Preferred transfected host cells are *E. coli* cells. The most preferred *E. coli* host cells are *E.coli*/pRS113 and *E. coli*/pRS103.

Host cells wherein a DNA expression vector encodes HSGluR1 receptor or HSGluR2 receptor are also provided. Preferably, the DNA vector comprises SEQ ID NO:2 or SEQ ID NO:4. Preferred host cells for expression of HSGluR1 and/or HSGluR2 are mammalian cells. Preferred mammalian cells for expression of HSGluR1 and/or HSGluR2 are COS-7 cells.

Specifically, host cells which have been transfected with a DNA expression vector which expresses functional HSGluR1 receptor or functional HSGluR2 receptor are provided. Host cells which have been transfected with a DNA expression vector which expresses functional HSGluR2/HSGluR1 complex are also provided. Host cells which have been transfected with a DNA expression vector which expresses functional HSGluR2/HSGluR1 complex and which further comprise a DNA vector which encodes a GluR3 receptor are provided. Host cells which comprise a DNA vector which encodes HSGluR2 receptor which further comprises a vector encoding GluR1 receptor is also provided. Likewise, host cells which comprise a DNA vector which encodes HSGluR2 receptor which further comprises a vector encoding GluR3 receptor are provided. Moreover, host cells which comprise a DNA expression vector which expresses HSGluR2 receptor, and which further comprise a DNA vector which encodes a GluR1 receptor, and which further comprise a DNA vector which encodes a GluR3 receptor are also provided. Also, host cells which have been transfected with a DNA expression vector which expresses a functional HSGluR1 receptor and which further comprise a DNA vector which encodes a functional GluR2 receptor are provided. Host cells which have been transfected with an DNA expression vector which expresses a functional HSGluR1 receptor, and further comprise a DNA vector which encodes a functional GluR2 receptor, and further comprise a DNA vector which encodes a functional GluR3 receptor are also provided.

Additionally, the invention provides a method for identifying DNA homologous to a probe of the present invention, which comprises contacting test nucleic acid with the probe under hybridizing conditions and identifying test nucleic acids which hybridize. The preferred method utilizes SEQ ID NO:5, SEQ ID NO:6, SEQ ID NO:7, SEQ ID NO: 8, SEQ ID NO: 9, or SEQ ID NO:10 as probes.

Assays utilizing the compounds provided by the present invention are also provided. These assays determine whether a substance interacts with or affects HSGluR1 or HSGluR2, said assay comprising introducing the substance and functional HSGluR1 and/or functional HSGluR2 into an acceptable medium, and monitoring interaction by physically detectable means, thereby identifying those substances which interact with or affect HSGluR1 or HSGluR2. Preferred assays include those which utilize both HSGluR2 and HSGluR1 receptor, and which results in a functional HSGluR2/HSGluR1 complex. Assays provided include those which utilize both functional HSGluR2/HSGluR1 complex and GluR3 receptor. Other assays include those which utilize HSGluR2 and GluR1 receptor or HSGluR2 and GluR3 receptor. Another assay utilizes HSGluR2 and both a GluR1 receptor and a GluR3 receptor. Other assays utilize HSGluR1 and GLuR2 receptors or HSGluR1, GluR2, and GluR3 receptors.

Preferably, the physically detectable means is selected from 1) competing with labeled glutamate, 2) interacting with glutaminergic ligand or 3) generating ion flow. A preferred assay is an oocyte assay system. A most preferred competition assay system utilizes radioactively-labeled glutamate. A most preferred oocyte assay system utilizes sense mRNA.

The invention also provides a method for constructing a host cell capable of expressing a nucleic acid compound which encodes a compound which comprises SEQ ID NO:1, SEQ ID NO:3, or functional equivalents thereof, said method comprising transfecting a host cell with a DNA vector which comprises said nucleic acid compound. A preferred method utilizes mammalian cells as the host cells. A more preferred method further utilizes COS-7 mammalian cells. A more preferred method further comprises a DNA vector. In a most preferred method, a DNA vector comprises SEQ ID NO:2, SEQ ID NO:4, or functional equivalents thereof.

Additionally, a method for expressing a nucleic acid sequence which encodes SEQ ID NO:1 and/or SEQ ID NO:3 in a host cell is provided. The method comprises transfecting host cells with nucleic acids of the present invention and culturing the transfected host cells under conditions suitable for gene expression. A preferred method utilizes mammalian cells as the host cells. A more preferred method utilizes COS-7 mammalian cells. An even more preferred method utilizes a DNA vector. A most preferred method utilizes both COS-7 cells and a DNA vector comprising SEQ ID NO:2, SEQ ID NO:4, or functional equivalents thereof.

The methods provided also include those which utilize oocytes as the host cell. Preferrably, a method utilizing oocytes also utilizes sense mRNA.

For purposes of clarity and as an aid in understanding the invention, as disclosed and claimed herein, the following items are defined below.

"Functional HSGluR1" - A compound comprising SEQ ID NO:1 which, when alone or combined with another glutamate receptor, is capable of generating ion flow, binding glutamate, interacting with glutaminergic ligand, or performing in a manner consistent with a glutamate receptor.

"Functional HSGluR2" - A compound comprising SEQ ID NO:3 which, when alone or combined with another glutamate receptor, is capable of generating ion flow, binding glutamate, interacting with glutaminergic ligand, or performing in a manner consistent with a glutamate receptor.

"GluR1 receptor" - The amino acid sequence commonly associated with the rat ionoptropic glutamate receptor 1.

"GluR2 receptor" - The amino acid sequence commonly associated with the rat ionotropic glutamate receptor 2.

"GluR3 receptor" - The amino acid sequence commonly associated with the rat ionotropic glutamate receptor 3.

"HSGluR1 receptor" - The compound having an amino acid sequence SEQ ID NO:1 or functional equilvents thereof.

"HSGluR2 receptor" - The compound having amino acid sequence SEQ ID NO:3 or functional equivalents thereof.

"mRNA" - RNA which has been transcribed either *in vivo* or *in vitro,* including, for example, RNA transcripts prepared *in vitro* via transcription of coding sequences of DNA by RNA polymerase.

"Part of SEQ ID NO:1" - A sequence containing at least 6 consecutive amino acid residues or more and that corresponds to a sequence contained in SEQ ID NO:1.

"Part of SEQ ID NO:3" - A sequence containing at least 6 consecutive amino acid residues or more and that corresponds to a sequence contained in SEQ ID NO:3.

"Physically detectable" - Any information which has been presented in humanly recognizable form, with or without the aid of intervening interpretation. For example, electrophysiological, chemical or biochemical data is considered within the realm of physically detectable information.

"Primer" - A nucleic acid fragment or its reverse complement which functions as template for enzymatic or synthetic elongation.

"Probe" - A nucleic acid compound or a fragment thereof, or their reverse complement, either of which is used to hybridize to other nucleic acids.

"SEQ ID NO:1 and functional equivalents thereof" - SEQ ID NO:1 and conservative alterations of the amino acid sequence of SEQ ID NO:1, wherein the conservative alterations result in a compound which exhibits substantially the same biological, biochemical, physical and structural qualities of SEQ ID NO:1.

"SEQ ID NO:3 and functional equivalents thereof" - SEQ ID NO:3 and conservative alterations of the amino acid sequence of SEQ ID NO:3, wherein the conservative alterations result in a compound which exhibits substantially the same biological, biochemical, physical and structural qualities of SEQ ID:3.

"SEQ ID NO:2" - a DNA sequence which encodes SEQ ID NO:1.

"SEQ ID NO:4" - a DNA sequence which encodes SEQ ID NO:3.

"SEQ ID NO:5" - The DNA sequence: ATG CAG CAC ATT TTT GCC TTC TTC TGC ACC GGT TTC CTA GGC GCG GTA GTA GGT GCC AAT. This segment includes bases 1 through 60 of SEQ ID NO:2, counting from the 5' end.

"SEQ ID NO:6" - The DNA sequence: TTT GCT TTG TCG CAA CTC ACA GAG CCC CCG AAG CTG CTC CCC CAG ATT GAT ATT GTG AAC. This segment includes bases 130-189 of SEQ ID NO:2, counting from the 5' end.

"SEQ ID NO:7" - The DNA sequence: CAA TCG ATT CCT TGC ATG AGC CAC AGT TCA GGG ATG CCC TTG GGA GCC ACG GGA TTG TAA. This segment includes bases 2662-2718 of SEQ ID NO:2, with a TAA stop codon added at the 3' end.

"SEQ ID NO:8" - The DNA sequence: ATGCAAAAGA TTATGCATAT TTCTGTCCTC CTTTCTCCTG TTTTATGGGG ACTGATTTTT. This segment includes bases 1 through 60 of SEQ ID NO:4, counting from the 5' end.

"SEQ ID NO:9" - The DNA sequence: GTAGG GATGG TTCAGTTTTC CACTTCGGAG TTCAGACTGA CACCCCACAT CGACAATTTG. This segment includes bases 136 through 195 of SEQ ID NO:2, counting from the 5' end.

"SEQ ID NO:10" - The DNA sequence: AATTTTGCAA CTTATAAGGA AGGTTACAAC GTATATGGCA TCGAAAGTGT TAAAATTTAA. This segment includes bases 2593 through 2649 of SEQ ID NO:4, with a TAA stop codon added at the 3' end.

"Transfection" - Any transfer of nucleic acid into a host cell, with or without integration of said nucleic acid into genome of said host cell.

The present invention provides an amino acid compound which comprises the isolated amino acid sequence SEQ ID NO:1 and functional equivalents thereof. The preferred amino acid compound is SEQ ID NO:1, which is the following sequence of amino acids:

Met Gln His Ile Phe Ala Phe Phe Cys Thr Gly Phe Leu Gly Ala Val
1               5                   10                  15

Val Gly Ala Asn Phe Pro Asn Asn Ile Gln Ile Gly Gly Leu Phe Pro
            20                  25                  30

Asn Gln Gln Ser Gln Glu His Ala Ala Phe Arg Phe Ala Leu Ser Gln
        35                  40                  45

Leu Thr Glu Pro Pro Lys Leu Leu Pro Gln Ile Asp Ile Val Asn Ile
    50                  55                  60

Ser Asp Ser Phe Glu Met Thr Tyr Arg Phe Cys Ser Gln Phe Ser Lys
65                  70                  75                  80

Gly Val Tyr Ala Ile Phe Gly Phe Tyr Glu Arg Arg Thr Val Asn Met
                85                  90                  95

Leu Thr Ser Phe Cys Gly Ala Leu His Val Cys Phe Ile Thr Pro Ser
            100                 105                 110

Phe Pro Val Asp Thr Ser Asn Gln Phe Val Leu Gln Leu Arg Pro Glu
        115                 120                 125

Leu Gln Asp Ala Leu Ile Ser Ile Ile Asp His Tyr Lys Trp Gln Lys
    130                 135                 140

Phe Val Tyr Ile Tyr Asp Ala Asp Arg Gly Leu Ser Val Leu Gln Lys
145                 150                 155                 160

Val Leu Asp Thr Ala Ala Glu Lys Asn Trp Gln Val Thr Ala Val Asn
                165                 170                 175

Ile Leu Thr Thr Thr Glu Glu Gly Tyr Arg Met Leu Phe Gln Asp Leu
            180                 185                 190

Glu Lys Lys Lys Glu Arg Leu Val Val Val Asp Cys Glu Ser Glu Arg
        195                 200                 205

Leu Asn Ala Ile Leu Gly Gln Ile Ile Lys Leu Glu Lys Asn Gly Ile
    210                 215                 220

Gly Tyr His Tyr Ile Leu Ala Asn Leu Gly Phe Met Asp Ile Asp Leu
225                 230                 235                 240

Asn Lys Phe Lys Glu Ser Gly Ala Asn Val Thr Gly Phe Gln Leu Val
                245                 250                 255

Asn Tyr Thr Asp Thr Ile Pro Ala Lys Ile Met Gln Gln Trp Lys Asn
            260                 265                 270

Ser Asp Ala Arg Asp His Thr Arg Val Asp Trp Lys Arg Pro Lys Tyr
        275                 280                 285

Thr Ser Ala Leu Thr Tyr Asp Gly Val Lys Val Met Ala Glu Ala Phe
    290                 295                 300

Gln Ser Leu Arg Arg Gln Arg Ile Asp Ile Ser Arg Arg Gly Asn Ala
305                 310                 315                 320

Gly Asp Cys Leu Ala Asn Pro Ala Val Pro Trp Gly Gln Gly Ile Asp
                325                 330                 335

Ile Gln Arg Ala Leu Gln Gln Val Arg Phe Glu Gly Leu Thr Gly Asn
            340                 345                 350

Val Gln Phe Asn Glu Lys Gly Arg Arg Thr Asn Tyr Thr Leu His Val
        355                 360                 365

Ile Glu Met Lys His Asp Gly Ile Arg Lys Ile Gly Tyr Trp Asn Glu
    370                 375                 380

Asp Asp Lys Phe Val Pro Ala Ala Thr Asp Ala Gln Ala Gly Gly Asp
385                 390                 395                 400

Asn Ser Ser Val Gln Asn Arg Thr Tyr Ile Val Thr Thr Ile Leu Glu
                405                 410                 415

Asp Pro Tyr Val Met Leu Lys Lys Asn Ala Asn Gln Phe Glu Gly Asn
            420                 425                 430

Asp Arg Tyr Glu Gly Tyr Cys Val Glu Leu Ala Ala Glu Ile Ala Lys
        435                 440                 445

His Val Gly Tyr Ser Tyr Arg Leu Glu Ile Val Ser Asp Gly Lys Tyr
    450                 455                 460

Gly Ala Arg Asp Pro Asp Thr Lys Ala Trp Asn Gly Met Val Gly Glu
465                 470                 475                 480

Leu Val Tyr Gly Arg Ala Asp Val Ala Val Ala Pro Leu Thr Ile Thr
                485                 490                 495

Leu Val Arg Glu Glu Val Ile Asp Phe Ser Lys Pro Phe Met Ser Leu
            500                 505                 510

Gly Ile Ser Ile Met Ile Lys Lys Pro Gln Lys Ser Lys Pro Gly Val
        515                 520                 525

Phe Ser Phe Leu Asp Pro Leu Ala Tyr Glu Ile Trp Met Cys Ile Val
    530                 535                 540

Phe Ala Tyr Ile Gly Val Ser Val Val Leu Phe Leu Val Ser Arg Phe
545                 550                 555                 560

Ser Pro Tyr Glu Trp His Ser Glu Glu Phe Glu Glu Gly Arg Asp Gln
                565                 570                 575

Thr Thr Ser Asp Gln Ser Asn Glu Phe Gly Ile Phe Asn Ser Leu Trp
            580                 585                 590

Phe Ser Leu Gly Ala Phe Met Gln Gln Gly Cys Asp Ile Ser Pro Arg
        595                 600                 605

Ser Leu Ser Gly Arg Ile Val Gly Gly Val Trp Trp Phe Phe Thr Leu
    610                 615                 620

Ile Ile Ile Ser Ser Tyr Thr Ala Asn Leu Ala Ala Phe Leu Thr Val
625                 630                 635                 640

Glu Arg Met Val Ser Pro Ile Glu Ser Ala Glu Asp Leu Ala Lys Gln
                645                 650                 655

Thr Glu Ile Ala Tyr Gly Thr Leu Glu Ala Gly Ser Thr Lys Glu Phe
            660                 665                 670

Phe Arg Arg Ser Lys Ile Ala Val Phe Glu Lys Met Trp Thr Tyr Met
        675                 680                 685

Lys Ser Ala Glu Pro Ser Val Phe Val Arg Thr Thr Glu Glu Gly Met
    690                 695                 700

Ile Arg Val Arg Lys Ser Lys Gly Lys Tyr Ala Tyr Leu Leu Glu Ser
705                 710                 715                 720

Thr Met Asn Glu Tyr Ile Glu Gln Arg Lys Pro Cys Asp Thr Met Lys
                725                 730                 735

Val Gly Gly Asn Leu Asp Ser Lys Gly Tyr Gly Ile Ala Thr Pro Lys
            740                 745                 750

Gly Ser Ala Leu Arg Asn Pro Val Asn Leu Ala Val Leu Lys Leu Asn
        755                 760                 765

Glu Gln Gly Leu Leu Asp Lys Leu Lys Asn Lys Trp Trp Tyr Asp Lys
    770                 775                 780

Gly Glu Cys Gly Ser Gly Gly Gly Asp Ser Lys Asp Lys Thr Ser Ala
785                 790                 795                 800

Leu Ser Leu Ser Asn Val Ala Gly Val Phe Tyr Ile Leu Ile Gly Gly
                805                 810                 815

Leu Gly Leu Ala Met Leu Val Ala Leu Ile Glu Phe Cys Tyr Lys Ser
            820                 825                 830

Arg Ser Glu Ser Lys Arg Met Lys Gly Phe Cys Leu Ile Pro Gln Gln
        835                 840                 845

Ser Ile Asn Glu Ala Ile Arg Thr Ser Thr Leu Pro Arg Asn Ser Gly
    850                 855                 860

Ala Gly Ala Ser Ser Gly Gly Ser Gly Glu Asn Gly Arg Val Val Ser
865                 870                 875                 880

His Asp Phe Pro Lys Ser Met Gln Ser Ile Pro Cys Met Ser His Ser
                885                 890                 895

Ser Gly Met Pro Leu Gly Ala Thr Gly Leu
            900                 905

The present invention also provides compounds which comprises the amino acid sequence SEQ ID NO:3 and functional equivalents thereof. The preferred amino acid compound is SEQ ID NO:3, which is the following sequence of amino acids:

Met Gln Lys Ile Met His Ile Ser Val Leu Leu Ser Pro Val Leu Trp
1               5                   10                  15

Gly Leu Ile Phe Gly Val Ser Ser Asn Ser Ile Gln Ile Gly Gly Leu
            20                  25                  30

Phe Pro Arg Gly Ala Asp Gln Glu Tyr Ser Ala Phe Arg Val Gly Met
        35                  40                  45

Val Gln Phe Ser Thr Ser Glu Phe Arg Leu Thr Pro His Ile Asp Asn
    50                  55                  60

Leu Glu Val Ala Asn Ser Phe Ala Val Thr Asn Ala Phe Cys Ser Gln
65                  70                  75                  80

Phe Ser Arg Gly Val Tyr Ala Ile Phe Gly Phe Tyr Asp Lys Lys Ser
                85                  90                  95

Val Asn Thr Ile Thr Ser Phe Cys Gly Thr Leu His Val Ser Phe Ile
            100                 105                 110

Thr Pro Ser Phe Pro Thr Asp Gly Thr His Pro Phe Val Ile Gln Met
        115                 120                 125

Arg Pro Asp Leu Lys Gly Ala Leu Leu Ser Leu Ile Glu Tyr Tyr Gln
    130                 135                 140

Trp Asp Lys Phe Ala Tyr Leu Tyr Asp Ser Asp Arg Gly Leu Ser Thr
145                 150                 155                 160

Leu Gln Ala Val Leu Asp Ser Ala Ala Glu Lys Lys Trp Gln Val Thr
                165                 170                 175

Ala Ile Asn Val Gly Asn Ile Asn Asn Asp Lys Lys Asp Glu Met Tyr
            180                 185                 190

Arg Ser Leu Phe Gln Asp Leu Glu Leu Lys Lys Glu Arg Arg Val Ile
        195                 200                 205

Leu Asp Cys Glu Arg Asp Lys Val Asn Asp Ile Val Asp Gln Val Ile
    210                 215                 220

Thr Ile Gly Lys His Val Lys Gly Tyr His Tyr Ile Ile Ala Asn Leu
225                 230                 235                 240

Gly Phe Thr Asp Gly Asp Leu Leu Lys Ile Gln Phe Gly Gly Ala Asn
                245                 250                 255

Val Ser Gly Phe Gln Ile Val Asp Tyr Asp Asp Ser Leu Val Ser Lys
            260                 265                 270

Phe Ile Glu Arg Trp Ser Thr Leu Glu Glu Lys Glu Tyr Pro Gly Ala
        275                 280                 285

His Thr Thr Thr Ile Lys Tyr Thr Ser Ala Leu Thr Tyr Asp Ala Val
    290                 295                 300

Gln Val Met Thr Glu Ala Phe Arg Asn Leu Arg Lys Gln Arg Ile Glu
305                 310                 315                 320

Ile Ser Arg Arg Gly Asn Ala Gly Asp Cys Leu Ala Asn Pro Ala Val
                325                 330                 335

Pro Trp Gly Gln Gly Val Glu Ile Glu Arg Ala Leu Lys Gln Val Gln
            340                 345                 350

Val Glu Gly Leu Ser Gly Asn Ile Lys Phe Asp Gln Asn Gly Lys Arg
        355                 360                 365

Ile Asn Tyr Thr Ile Asn Ile Met Glu Leu Lys Thr Asn Gly Pro Arg
    370                 375                 380

Lys Ile Gly Tyr Trp Ser Glu Val Asp Lys Met Val Val Thr Leu Thr
385                 390                 395                 400

Glu Leu Pro Ser Gly Asn Asp Thr Ser Gly Leu Glu Asn Lys Thr Val
                405                 410                 415

Val Val Thr Thr Ile Leu Glu Ser Pro Tyr Val Met Met Lys Lys Asn
            420                 425                 430

His Glu Met Leu Glu Gly Asn Glu Arg Tyr Glu Gly Tyr Cys Val Asp
        435                 440                 445

Leu Ala Ala Glu Ile Ala Lys His Cys Gly Phe Lys Tyr Lys Leu Thr
    450                 455                 460

Ile Val Gly Asp Gly Lys Tyr Gly Ala Arg Asp Ala Asp Thr Lys Ile
465                 470                 475                 480

Trp Asn Gly Met Val Gly Glu Leu Val Tyr Gly Lys Ala Asp Ile Ala
                485                 490                 495

Ile Ala Pro Leu Thr Ile Thr Leu Val Arg Glu Glu Val Ile Asp Phe
            500                 505                 510

Ser Lys Pro Phe Met Ser Leu Gly Ile Ser Ile Met Ile Lys Lys Pro
        515                 520                 525

Gln Lys Ser Lys Pro Gly Val Phe Ser Phe Leu Asp Pro Leu Ala Tyr
    530                 535                 540

Glu Ile Trp Met Cys Ile Val Phe Ala Tyr Ile Gly Val Ser Val Val
545                 550                 555                 560

Leu Phe Leu Val Ser Arg Phe Ser Pro Tyr Glu Trp His Thr Glu Glu
                565                 570                 575

Phe Glu Asp Gly Arg Glu Thr Gln Ser Ser Glu Ser Thr Asn Glu Phe
            580                 585                 590

Gly Ile Phe Asn Ser Leu Trp Phe Ser Leu Gly Ala Phe Met Arg Gln
        595                 600                 605

Gly Cys Asp Ile Ser Pro Arg Ser Leu Ser Gly Arg Ile Val Gly Gly
    610                 615                 620

Val Trp Trp Phe Phe Thr Leu Ile Ile Ile Ser Ser Tyr Thr Ala Asn
625                 630                 635                 640

Leu Ala Ala Phe Leu Thr Val Glu Arg Met Val Ser Pro Ile Glu Ser

                645                 650                 655

Ala Glu Asp Leu Ser Lys Gln Thr Glu Ile Ala Tyr Gly Thr Leu Asp
            660                 665                 670

Ser Gly Ser Thr Lys Glu Phe Phe Arg Arg Ser Lys Ile Ala Val Phe
        675                 680                 685

Asp Lys Met Trp Thr Tyr Met Arg Ser Ala Glu Pro Ser Val Phe Val
    690                 695                 700

Arg Thr Thr Ala Glu Gly Val Ala Arg Val Arg Lys Ser Lys Gly Lys
705                 710                 715                 720

Tyr Ala Tyr Leu Leu Glu Ser Thr Met Asn Glu Tyr Ile Glu Gln Arg
                725                 730                 735

Lys Pro Cys Asp Thr Met Lys Val Gly Gly Asn Leu Asp Ser Lys Gly
            740                 745                 750

Tyr Gly Ile Ala Thr Pro Lys Gly Ser Ser Leu Arg Thr Pro Val Asn
        755                 760                 765

Leu Ala Val Leu Lys Leu Ser Glu Gln Gly Val Leu Asp Lys Leu Lys
    770                 775                 780

Asn Lys Trp Trp Tyr Asp Lys Gly Glu Cys Gly Ala Lys Asp Ser Gly
785                 790                 795                 800

Ser Lys Glu Lys Thr Ser Ala Leu Ser Leu Ser Asn Val Ala Gly Val
                805                 810                 815

Phe Tyr Ile Leu Val Gly Gly Leu Gly Leu Ala Met Leu Val Ala Leu
            820                 825                 830

Ile Glu Phe Cys Tyr Lys Ser Arg Ala Glu Ala Lys Arg Met Lys Val
        835                 840                 845

Ala Lys Asn Ala Gln Asn Ile Asn Pro Ser Ser Ser Gln Asn Ser Gln
    850                 855                 860

Asn Phe Ala Thr Tyr Lys Glu Gly Tyr Asn Val Tyr Gly Ile Glu Ser
865                 870                 875                 880

Val Lys Ile.

Those in the art will recognize that some alterations of SEQ ID NO:1 or SEQ ID NO:3 will fail to change the function of the compound. For instance, some hydrophobic amino acids may be exchanged for other hydrophobic amino acids. Those altered compounds which confer the function of SEQ ID NO:1 or SEQ ID NO:3 in substantially the same manner as the exemplified compound are also included in the present invention.

Artisans will also recognize that these compounds can be synthesized by a number of different methods. All of the amino acid compounds of the invention can be made by chemical methods well known in the art, including solid phase peptide synthesis or recombinant methods. Both methods are described in U.S. Patent 4,617,149. Recombinant methods are preferred if a high yield is desired. A general method for the construction of any desired DNA sequence is provided in Brown *et al.,* 68 *Methods in Enzymology* 109 (1979).

Other routes of production are well known to those in the art. Expression in eucaryotic cells can be achieved via SEQ ID NO:2 and SEQ ID NO:4 for the amino acid compounds of SEQ ID NO:1 and SEQ ID NO:3, respectively. For example, the amino acid compounds can be produced in eucaryotic cells using SV40-derived expression vectors comprising DNA which encodes for SEQ ID NO:1 and/or SEQ ID NO:3. As is well known in the art, some viruses are also appropriate vectors. For example, the adenoviruses, the papovaviruses, the vaccinia viruses, the herpes viruses, and the baculoviruses, as well as vectors derived from these viruses, are useful. Such a method is described in U.S. Patent 4,775,624. Several alternate methods of expression are described in J. Sambrook, E.F. Fritsch & T.Maniatis, *Molecular Cloning: A Laboratory Manual* 16.3-17.44 (1989) and *Methods in Enzymology,* Vol. 185 (1990).

Other embodiments of the present invention are nucleic acid compounds which comprise nucleic acid sequences which encode all or part of SEQ ID NO:1, SEQ ID NO:3, or functional equivalents thereof. As artisans will recognize, the amino acid compounds of the invention can be encoded by a multitude of different nucleic acid sequences because most of the amino acids are encoded by more than one nucleic acid triplet. Because these alternate nucleic acid sequences would encode substantially the same amino acid sequence, the present invention further comprises these alternate nucleic acid sequences. Preferably, the nucleic acid compound is DNA or sense mRNA.

A most preferred embodiment of a DNA compound encoding the HSGluR1 compound has this sequence: 
ATG CAG CAC ATT TTT GCC TTC TTC TGC ACC GGT TTC CTA GGC GCG GTA
GTA GGT GCC AAT TTC CCC AAC AAT ATC CAG ATC GGG GGA TTA TTT CCA
AAC CAG CAG TCA CAG GAA CAT GCT GCT TTT AGA TTT GCT TTG TCG CAA
CTC ACA GAG CCC CCG AAG CTG CTC CCC CAG ATT GAT ATT GTG AAC ATC
AGC GAC AGC TTT GAG ATG ACC TAT AGA TTC TGT TCC CAG TTC TCC AAA
GGA GTC TAT GCC ATC TTT GGG TTT TAT GAA CGT AGG ACT GTC AAC ATG
CTG ACC TCC TTT TGT GGG GCC CTC CAC GTC TGC TTC ATT ACG CCG AGC
TTT CCC GTT GAT ACA TCC AAT CAG TTT GTC CTT CAG CTG CGC CCT GAA
CTG CAG GAT GCC CTC ATC AGC ATC ATT GAC CAT TAC AAG TGG CAG AAA
TTT GTC TAC ATT TAT GAT GCC GAC CGG GGC TTA TCC GTC CTG CAG AAA
GTC CTG GAT ACA GCT GCT GAG AAG AAC TGG CAG GTG ACA GCA GTC AAC
ATT TTG ACA ACC ACA GAG GAG GGA TAC CGG ATG CTC TTT CAG GAC CTG
 
GAG AAG AAA AAG GAG CGG CTG GTG GTG GTG GAC TGT GAA TCA GAA CGC
CTC AAT GCT ATC TTG GGC CAG ATT ATA AAG CTA GAG AAG AAT GGC ATC
GGC TAC CAC TAC ATT CTT GCA AAT CTG GGC TTC ATG GAC ATT GAC TTA
AAC AAA TTC AAG GAG AGT GGC GCC AAT GTG ACA GGT TTC CAG CTG GTG
AAC TAC ACA GAC ACT ATT CCG GCC AAG ATC ATG CAG CAG TGG AAG AAT
AGT GAT GCT CGA GAC CAC ACA CGG GTG GAC TGG AAG AGA CCC AAG TAC
ACC TCT GCG CTC ACC TAC GAT GGG GTG AAG GTG ATG GCT GAG GCT TTC
CAG AGC CTG CGG AGG CAG AGA ATT GAT ATA TCT CGC CGG GGG AAT GCT
GGG GAT TGT CTG GCT AAC CCA GCT GTT CCC TGG GGC CAA GGG ATC GAC
ATC CAG AGA GCT CTG CAG CAG GTG CGA TTT GAA GGT TTA ACA GGA AAC
GTG CAG TTT AAT GAG AAA GGA CGC CGG ACC AAC TAC ACG CTC CAC GTG
ATT GAA ATG AAA CAT GAC GGC ATC CGA AAG ATT GGT TAC TGG AAT GAA
GAT GAT AAG TTT GTC CCT GCA GCC ACC GAT GCC CAA GCT GGG GGC GAT
AAT TCA AGT GTT CAG AAC AGA ACA TAC ATC GTC ACA ACA ATC CTA GAA
GAT CCT TAT GTG ATG CTC AAG AAG AAC GCC AAT CAG TTT GAG GGC AAT
GAC CGT TAC GAG GGC TAC TGT GTA GAG CTG GCG GCA GAG ATT GCC AAG
CAC GTG GGC TAC TCC TAC CGT CTG GAG ATT GTC AGT GAT GGA AAA TAC
GGA GCC CGA GAC CCT GAC ACG AAG GCC TGG AAT GGC ATG GTG GGA GAG
CTG GTC TAT GGA AGA GCA GAT GTG GCT GTG GCT CCC TTA ACT ATC ACT
TTG GTC CGG GAA GAA GTT ATA GAT TTC TCC AAA CCA TTT ATG AGT TTG
GGG ATC TCC ATC ATG ATT AAA AAA CCA CAG AAA TCC AAG CCG GGT GTC
TTC TCC TTC CTT GAT CCT TTG GCT TAT GAG ATT TGG ATG TGC ATT GTT
TTT GCC TAC ATT GGA GTG AGT GTT GTC CTC TTC CTG GTC AGC CGC TTC
AGT CCC TAT GAA TGG CAC AGT GAA GAG TTT GAG GAA GGA CGG GAC CAG
ACA ACC AGT GAC CAG TCC AAT GAG TTT GGG ATA TTC AAC AGT TTG TGG
 
TTC TCC CTG GGA GCC TTC ATG CAG CAA GGA TGT GAC ATT TCT CCC AGG
TCC CTG TCT GGT CGC ATC GTT GGT GGC GTC TGG TGG TTC TTC ACC TTA
ATC ATC ATC TCC TCA TAT ACA GCC AAT CTG GCC GCC TTC CTG ACC GTG
GAG AGG ATG GTG TCT CCC ATT GAG AGT GCA GAG GAC CTA GCG AAG CAG
ACA GAA ATT GCC TAC GGG ACG CTG GAA GCA GGA TCT ACT AAG GAG TTC
TTC AGG AGG TCT AAA ATT GCT GTG TTT GAG AAG ATG TGG ACA TAC ATG
AAG TCA GCA GAG CCA TCA GTT TTT GTG CGG ACC ACA GAG GAG GGG ATG
ATT CGA GTG AGG AAA TCC AAA GGC AAA TAT GCC TAC CTC CTG GAG TCC
ACC ATG AAT GAG TAC ATT GAG CAG CGG AAA CCC TGT GAC ACC ATG AAG
GTG GGA GGT AAC TTG GAT TCC AAA GGC TAT GGC ATT GCA ACA CCC AAG
GGG TCT GCC CTG AGA AAT CCA GTA AAC CTG GCA GTG TTA AAA CTG AAC
GAG CAG GGG CTT TTG GAC AAA TTG AAA AAC AAA TGG TGG TAC GAC AAG
GGC GAG TGC GGC AGC GGG GGA GGT GAT TCC AAG GAC AAG ACA AGC GCT
CTG AGC CTC AGC AAT GTG GCA GGC GTG TTC TAC ATC CTG ATC GGA GGA
CTT GGA CTA GCC ATG CTG GTT GCC TTA ATC GAG TTC TGC TAC AAA TCC
CGT AGT GAA TCC AAG CGG ATG AAG GGT TTT TGT TTG ATC CCA CAG CAA
TCC ATC AAC GAA GCC ATA CGG ACA TCG ACC CTC CCC CGC AAC AGC GGG
GCA GGA GCC AGC AGC GGC GGC AGT GGA GAG AAT GGT CGG GTG GTC AGC
CAT GAC TTC CCC AAG TCC ATG CAA TCG ATT CCT TGC ATG AGC CAC AGT
TCA GGG ATG CCC TTG GGA GCC ACG GGA TTG
This is the sequence identified as SEQ ID NO:2.

A second most preferred embodiment of a DNA compound encoding in the HSGluR2 compound has this sequence: 
ATGCAAAAGA TTATGCATAT TTCTGTCCTC CTTTCTCCTG TTTTATGGGG ACTGATTTTT 60
GGTGTCTCTT CTAACAGCAT ACAGATAGGG GGGCTATTTC CTAGGGGCGC CGATCAAGAA 120
TACAGTGCAT TTCGAGTAGG GATGGTTCAG TTTTCCACTT CGGAGTTCAG ACTGACACCC 180
CACATCGACA ATTTGGAGGT GGCAAACAGC TTCGCAGTCA CTAATGCTTT CTGCTCCCAG 240
TTTTCGAGAG GAGTCTATGC TATTTTTGGA TTTTATGACA AGAAGTCTGT AAATACCATC 300
ACATCATTTT GCGGAACACT CCACGTCTCC TTCATCACTC CCAGCTTCCC AACAGATGGC 360
ACACATCCAT TTGTCATTCA GATGAGACCC GACCTCAAAG GAGCTCTCCT TAGCTTGATT 420
GAATACTATC AATGGGACAA GTTTGCATAC CTCTATGACA GTGACAGAGG CTTATCAACA 480
CTGCAAGCTG TGCTGGATTC TGCTGCTGAA AAGAAATGGC AAGTGACTGC TATCAATGTG 540
GGAAACATTA ACAATGACAA GAAAGATGAG ATGTACCGAT CACTTTTTCA AGATCTGGAG 600
TTAAAAAAGG AACGGCGTGT AATTCTGGAC TGTGAAAGGG ATAAAGTAAA CGACATTGTA 660
GACCAGGTTA TTACCATTGG AAAACATGTT AAAGGGTACC ACTACATCAT TGCAAATCTG 720
GGATTTACTG ATGGAGACCT ATTAAAAATC CAGTTTGGAG GTGCAAATGT CTCTGGATTT 780
CAGATAGTGG ACTATGATGA TTCGTTGGTA TCTAAATTTA TAGAAAGATG GTCAACACTG 840
GAAGAAAAAG AATACCCTGG AGCTCACACA ACAACAATTA AGTATACTTC TGCTCTGACC 900
TATGATGCCG TTCAAGTGAT GACTGAAGCC TTCCGCAACC TAAGGAAGCA AAGAATTGAA 960
ATCTCCCGAA GGGGGAATGC AGGAGACTGT CTGGCAAACC CAGCAGTGCC CTGGGGACAA 1020
GGTGTAGAAA TAGAAAGGGC CCTCAAACAG GTTCAGGTTG AAGGTCTCTC AGGAAATATA 1080
AAGTTTGACC AGAATGGAAA AAGAATAAAC TATACAATTA ACATCATGGA GCTCAAAACT 1140
AATGGGCCCC GGAAGATTGG CTACTGGAGT GAAGTGGACA AAATGGTTGT TACCCTTACT 1200
GAGCTCCCTT CTGGAAATGA CACCTCTGGG CTTGAGAATA AGACTGTTGT TGTCACCACA 1260
ATTTTGGAAT CTCCGTATGT TATGATGAAG AAAAATCATG AAATGCTTGA AGGCAATGAG 1320
CGCTATGAGG GCTACTGTGT TGACCTGGCT GCAGAAATCG CCAAACATTG TGGGTTCAAG 1380
TACAAGTTGA CAATTGTTGG TGATGGCAAG TATGGGGCCA GGGATGCAGA CACGAAAATT 1440
TGGAATGGGA TGGTTGGAGA ACTTGTATAT GGGAAAGCTG ATATTGCAAT TGCTCCATTA 1500
 
ACTATTACCC TTGTGAGAGA AGAGGTGATT GACTTCTCAA AGCCCTTCAT GAGCCTCGGG 1560

ATATCTATCA TGATCAAGAA GCCTCAGAAG TCCAAACCAG GAGTGTTTTC CTTTCTTGAT 1620

CCTTTAGCCT ATGAGATCTG GATGTGCATT GTTTTTGCCT ACATTGGGGT CAGTGTAGTT 1680

TTATTCCTGG TCAGCAGATT TAGCCCCTAC GAGTGGCACA CTGAGGAGTT TGAAGATGGA 1740

AGAGAAACAC AAAGTAGTGA ATCAACTAAT GAATTTGGGA TTTTTAATAG TCTCTGGTTT 1800

TCCTTGGGTG CCTTTATGCG GCAAGGATGC GATATTTCGC CAAGATCCCT CTCTGGGCGC 1860

ATTGTTGGAG GTGTGTGGTG GTTCTTTACC CTGATCATAA TCTCCTCCTA CACGGCTAAC 1920

TTAGCTGCCT TCCTGACTGT AGAGAGGATG GTGTCTCCCA TCGAAAGTGC TGAGGATCTT 1980

TCTAAGCAAA CAGAAATTGC TTATGGAACA TTAGACTCTG GCTCCACTAA AGAGTTTTTC 2040

AGGAGATCTA AAATTGCAGT GTTTGATAAA ATGTGGACCT ACATGCGGAG TGCGGAGCCC 2100

TCTGTGTTTG TGAGGACTAC GGCCGAAGGG GTGGCTAGAG TGCGGAAGTC CAAAGGGAAA 2160

TATGCCTACT TGTTGGAGTC CACGATGAAC GAGTACATTG AGCAAAGGAA GCCTTGCGAC 2220

ACCATGAAAG TTGGTGGAAA CCTGGATTCC AAAGGCTATG GCATCGCAAC ACCTAAAGGA 2280

TCCTCATTAA GAACCCCAGT AAATCTTGCA GTATTGAAAC TCAGTGAGCA AGGCGTCTTA 2340

GACAAGCTGA AAAACAAATG GTGGTACGAT AAAGGTGAAT GTGGAGCCAA GGACTCTGGA 2400

AGTAAGGAAA AGACCAGTGC CCTCAGTCTG AGCAACGTTG CTGGAGTATT CTACATCCTT 2460

GTCGGGGGCC TTGGTTTGGC AATGCTGGTG GCTTTGATTG AGTTCTGTTA CAAGTCAAGG 2520

GCCGAGGCGA AACGAATGAA GGTGGCAAAG AATGCACAGA ATATTAACCC ATCTTCCTCG 2580

CAGAATTCAC AGAATTTTGC AACTTATAAG GAAGGTTACA ACGTATATGG CATCGAAAGT 2640

GTTAAAATT. 2649
This is the sequence identified as SEQ ID NO:4.

*E. coli*/pRS103, which contains a cloning vector comprising SEQ ID NO:2, was deposited and made part of the stock culture collections of the Northern Regional Research Laboratories (NRRL), Agricultural Research Service, U.S. Department of Agriculture, Peoria, Illinois, 61604 on April 22, 1992, under the accession number NRRL B-18967. SEQ ID NO:2 can be isolated from the plasmid, for example, as a 4.2 kb EcoR1/Kpn1 restriction fragment. Other fragments are also useful in obtaining SEQ ID NO:2.

*E. coli*/pRS113, which contains a cloning vector comprising SEQ ID NO:4, was deposited and made part of the stock culture collection of the Northern Regional Research Laboratories (NRRL), Agricultural Research Service, U.S. Department of Agriculture, Peoria, Illinois, 61604 on April 22, 1992, under the accession number NRRL B-18968. SEQ ID NO:4 can be isolated from the plasmid, for example, as an approximately 2.9 kb AlwnI/SalI restriction fragment. Other fragments are also useful in obtaining SEQ ID NO:4.

Additionally, the DNA sequences can be synthesized using automated DNA synthesizers, such as the ABS (Applied Biosystems, 850 Lincoln Centre Drive, Foster City, CA 94404) 380B DNA synthesizer. The DNA sequences can also be generated by the polymerase chain reaction (PCR) as described in U.S. Patent No. 4,889,818.

Because those in the art will recognize that many vectors are available for expression and cloning, those expression and cloning vectors which comprise nucleic acids which encode SEQ ID NO:1, SEQ ID NO: 3, or functional equivalents thereof are included in the present invention. The preferred nucleic acid vectors are those which are DNA. Most preferred are DNA vectors which comprise the DNA sequence which is SEQ ID NO:2 or SEQ ID NO:4. The DNA vectors most preferred are plasmid pRS103 and plasmid pRS113.

Restriction fragments of these vectors are also provided. The preferred fragments of pRS103 are the 4.2 kb EcoR1/Kpn1 restriction fragment and the 2.8 kb EcoRI/ClaI restriction fragment. The preferred fragments of pRS113 are the approximately 2.9 kb AlwnI/SalI restriction fragment and the approximately 2.9 kb EcoRI restriction fragment.

DNA vectors which further comprise a promoter positioned to drive expression of HSGluR1 receptor or HSGluR2 receptor are also provided. Preferred DNA expression vectors are those wherein the promoter functions in mammalian cells. More preferred DNA expression vectors are those wherein the promoter functions in COS-7 cells. Most preferred COS-7 DNA expression vectors comprise SEQ ID NO:2 or SEQ ID NO:4.

Plasmid pRS103 may be isolated from the deposited *E. coli*/pRS103, using an ordinary cesium chloride DNA isolation procedure. Similarly, plasmid pRS113 may be isolated from the deposited *E.coli*/pRS113, using an ordinary cesium chloride DNA isolation procedure. These plasmids can be readily utilized to construct expression vectors which produce HSGluR1 receptors or HSGluR2 receptors in a variety of organisms and cell lines, including, for example, CV1 cells, COS cells, CHO cells, *E. coli,* Sf9 (as host for baculovirus), *Pichia* and *Saccharomycetes.* The current literature contains techniques for constructing expression vectors and for transfecting host cells. For example, Sambrook *et al., Molecular Cloning: A* *Laboratory Manual* Chapters 16 and 17 (1989), explains these techniques.

The construction protocols discussed in Sambrook *et al.* can be followed to construct analogous vectors for other organisms merely by substituting, if necessary, the appropriate regulatory elements using techniques well known to artisans. Promoters which may be used, for example, are the thymidine kinase promoter, the metallothionin promoter or various viral and immunoglobulin promoters.

The DNA compounds of the present invention also include primers or probes. Nucleic acid compounds of at least 17 base pairs which encode all or a part of SEQ ID NO:1 or SEQ ID NO:3 are included in the present invention. DNA is the preferred nucleic acid used as a probe or primer. Most preferred DNA compounds useful as probes or primers are: SEQ ID NO:5, SEQ ID NO:6, SEQ ID NO:7, SEQ ID NO:8, SEQ ID NO:9, and SEQ ID NO:10. Those in the art will recognize the techniques associated with probes and primers as well known. Any sequence of at least 17 consecutive base pairs in length of the nucleic acids of the present invention may be used to screen any other nucleic acid. For example, all or part of SEQ ID NO:5 and all or part of the reverse complement of SEQ ID NO:7 may be used to hybridize to the terminal ends of the coding sequence. Then, through PCR amplification, the full length sequence may be generated. The full length sequence can be subsequently subcloned into any vector of choice.

Alternatively, SEQ ID NO:5, SEQ ID NO:6, SEQ ID NO:7, SEQ ID NO:8, SEQ ID NO:9, or SEQ ID NO:10 may be radioactively labeled at the 5' end in order to screen cDNA libraries by conventional means. Furthermore, any piece of HSSluR1 DNA or HSGluR2 DNA which has been bound to a filter may be flooded with total mRNA transcripts, in order to then reverse-transcribe the mRNA transcripts which bind.

Primers and probes may be obtained by means well known in the art. For example, once pRS103 or pRS113 is isolated, restriction enzymes and subsequent gel separation may be used to isolate the fragment of choice.

Host cells which harbor the nucleic acids provided by the present invention are also provided. For example, oocytes which comprise nucleic acids of the present invention are provided. Oocytes wherein the nucleic acid is DNA are preferred. Also preferred are oocytes wherein the nucleic acid harbored is sense mRNA.

Furthermore, oocytes which harbor nucleic acids encoding functional HSGluR1 receptor or functional HSGluR2 receptor are provided. Oocytes which harbor nucleic acids capable of expressing functional HSGluR2/HSGluR1 complex are also provided by the present invention. Oocytes which, in addition to harboring nucleic acids capable of expressing functional HSGluR2/HSGluR1 complex, further harbor nucleic acids capable of expressing GluR3 receptor are also provided.

Oocytes which, in addition to harboring nucleic acids capable of expressing functional HSGluR1 receptor, further harbors nucleic acids capable of expressing functional GluR2 receptor are provided. Oocytes which, in addition to harboring nucleic acids capable of expressing functional HSGluR1 receptor, also harbors nucleic acids capable of expressing functional GluR2 receptor and also harbors nucleic acids capable of expressing functional GluR3 receptor are also provided. Most preferred oocytes of the present invention are those which harbor sense mRNA.

Moreover, oocytes which comprise vectors encoding functional HSGluR2 and which further comprise vectors encoding GluR1 receptor are provided. Likewise, oocytes which comprise vectors encoding functional HSGluR2 receptor and which further comprise vectors encoding GluR3 receptor are provided. Furthermore, oocytes which comprise vectors encoding functional HSGluR2 receptor, along with vectors encoding GluR1 receptor and vectors encoding GluR3 receptor are also provided. Most preferred oocytes of the present invention are those which harbor sense mRNA.

Host cells provided also include those comprising a nucleic acid vector which encodes SEQ ID NO:1 or SEQ ID NO:3. Preferred are those host cells wherein the nucleic acid vector is DNA. Most preferred are host cells wherein the DNA vector comprises the DNA sequence SEQ ID NO:2 or SEQ ID NO:4. Preferred host cells include *E.coli* cells. The most preferred *E.coli* cell is one transfected with plasmid pRS103 or plasmid pRS113.

Host cells which are transfected with a DNA expression vector which encodes HSGluR1 receptor or HSGluR2 receptor are also provided. Preferably, the DNA vector comprises SEQ ID NO:2 or SEQ ID NO:4. Preferred host cells for expression of functional receptors are mammalian cells. Preferred mammalian cells for expression of HSGluR1 or HSGluR2 are COS-7 cells.

Host cells which are transfected with DNA expression vectors encoding HSGluR2/HSGluR1 complex are also provided. Host cells which have been transfected with a DNA expression vector which expresses functional HSGluR2/HSGluR1 complex and which further comprise a vector which encodes a GluR3 receptor are also provided. Host cells which comprise a DNA expression vector encoding HSGluR2 and which further comprise a vector encoding GluR1 receptor are also part of the invention.

Furthermore, host cells which comprise a DNA expression vector encoding HSGluR1 or HSGluR2 and which further comprise a vector encoding GluR3 receptor are also provided. Host cells which (a) have been transfected with an DNA expression vector which expresses a functional HSGluR1 receptor, and (b) further comprise a DNA vector which encodes a functional GluR2 receptor, and (c) further comprise a DNA vector which encodes a functional GluR3 receptor are also provided. Host cells which (a) have been transfected with a DNA expression vector which expresses HSGluR2 receptor, and (b) further comprise a vector which encodes a GluR1 receptor, and (c) further comprise a vector which encodes a GluR3 receptor are also provided. Wigler M. *et al.,* 16 *Cell* 777 (1979), describe such a cotransfection procedure.

Oocytes harboring foreign nucleic acids can be constructed according to the procedures described in Lübbert, *et al.* 84 *Proc. Nat . Acad. Sci.* 4332 (1987) and Berger, *Methods in Enzymology,* Vol. 152 (1987). Other host cell transfection procedures are well known in the art. Nucleic acids which encode HSGluR1 can be obtained from NRRL, under accession number B-18967. Nucleic acids which encode HSGluR2 can be obtained from NRRL, under accession number B-18968. Nucleic acids which encode GluR1, GluR2, and GluR3 can be obtained according to Heinemann S. *et al.,* PCT publication WO91/06648 (1992).

Additionally, the invention provides a method for identifying DNA homologous to a probe of the present invention, which comprises contacting test nucleic acid with the probe under hybridizing conditions and identifying those nucleic acids which hybridize. The preferred probes for use in this method are SEQ ID NO:5, SEQ ID NO:6, SEQ ID NO:7, SEQ ID NO:8, SEQ ID NO:9,and SEQ ID NO:10. Hybridization techniques are well known in the art. Sambrook *et al., Molecular* *Cloning:* *A Laboratory Manual* 11 (1989) describe such procedures.

Assays utilizing the compounds provided by the present invention are also provided. Assays provided include a method for determining whether a substance interacts with or affects HSGluR1 or HSGluR2, said method comprising introducing said substance and functional HSGluR1 or functional HSGluR2 into an acceptable medium, and monitoring interaction by physically detectable means, thereby identifying those substances which interact with or affect HSGluR1 or HSGluR2. Still other assays further utilize a HSGluR1 receptor or a HSGluR2 receptor. Other assays utilize both functional HSGluR2/HSGluR1 complex and GluR3. Assays also include those which utilize both HSGluR2 and GluR1 receptors. Likewise, assays include those which utilize HSGluR2 and GluR3 receptors. Another assay utilizes HSGluR2, GluR1 and GluR3. Similarly, assays also include these which utilize both HSGluR1 and GluR2 receptors or HSGluR1 and GluR3 receptors. Another assay utilizes HSGluR1, GluR2 and GluR3.

Preferably, the physically detectable means is selected from 1) competing with labeled glutamate, 2) interacting with glutaminergic ligand or 3) generating ion flow. A most preferred competition assay utilizes radioactively labeled glutamate.

A preferred assay is an oocyte assay system. A most preferred oocyte assay system utilizes sense mRNA. Most preferred is an assay wherein the oocyte expression system utilizes sense mRNA.

The oocyte expression system can be constructed according to the procedure described in Lübbert, *et al.* 84 *Proc. Nat. Acad. Sci.* 4332 (1987) and Berger, *Methods in Enzymology,* Vol.152 (1987). The radiolabeled glutamate competition assay may be accomplished according to Foster and Fagg, 7 *Brain Res.* *Rev.* 103 (1984). The assay which measures ion flow may be accomplished according to Hamill O.P. et al., 391 (No. 2) Pflugers Archiv:European J. of Physiology, 85 (1981).

Artisans will recognize that competition assays results are described in terms of Kᵢ values and artisans realize that desirable Kᵢ values are dependent on the selectivity of the compound tested. For example, a compound with a Kᵢ which is less than 10 nM is generally considered an excellent candidate for drug therapy. However, a compound which has a lower affinity, but is selective for the particular receptor, may be an even better candidate. The present invention provides assays which indicate whether a substance has either a high affinity or low affinity to HSGluR1 receptor or HSGluR2 receptor.

The present invention also provides a method for constructing a host cell capable of expressing SEQ ID NO:1, SEQ ID NO:3, or functional equivalents thereof, said method comprising transfecting a host cell with a DNA vector that comprises a DNA sequence which encodes SEQ ID NO:1, SEQ ID NO:3, or functional equivalents thereof. A general method for the construction of any desired DNA sequence is provided in Brown *et al.,* 68 *Methods in Enzymology* 109 (1979).

A preferred method utilizes mammalian cells as host cells. Preferably, the mammalian cells utilized are for this method are COS-7 cells. An especially preferred method utilizes a DNA expression vector in COS-7 cells. An even more preferred method utilizes a DNA expression vector which comprises SEQ ID NO:2, SEQ ID NO:4, or functional equivalents thereof. Transfected host cells may be cultured under conditions well known to skilled artisans such that SEQ ID NO:1 is expressed, thus producing HSGluR1 activity in the recombinant host cell. Similarly, transfected host cells may be cultured such that SEQ ID NO:3 is expressed, thus producing HSGluR2 in the host cell.

Therefore, also provided by the present invention is a method for expressing a gene which encodes SEQ ID NO:1 and/or SEQ ID NO:3 in a transfected host cell, said method comprising culturing said transfected host cell under conditions suitable for gene expression. A preferred method utilizes mammalian cells. A most preferred method utilizes COS-7 cells. A more preferred method utilizes COS-7 cells as host cells for a DNA vector. A most preferred method utilizes COS-7 cells as host cells for a DNA vector comprising SEQ ID NO:2 or SEQ ID NO:4. Another method utilizes oocytes as the host cells. Methods wherein oocytes are utilized preferably expresses sense mRNA. Expression in host cells may be accomplished according to the procedures outlined in Sambrook *et al.,* *Molecular Cloning: A Laboratory Manual* 16-17 (1989).

The following are examples of the present invention:

### Example 1

### Growth of E. coli/pRS103

A lyophilized culture of *E. coli* containing plasmid pRS103 can be obtained from the American Type Culture Collection, Rockville, Maryland 20852, and inoculated into a suitable broth for the growth of *E.* *coli* using standard microbiological procedures.

The contents of a lyophil vial containing *E.coli*/pRS103 were transferred into 100 ml of sterile YT (tryptone-yeast extract) broth containing 100 µg/ml ampicillin in a one liter fermentation flask and shaken at 37°C on an orbital shaker at 250-300 rpm. After the optical density (OD, measured at 600 millimicrons) had reached approximately 1-2 OD, the bacterial cells were recovered and used for the isolation of plasmid pRS103 according to the procedures detailed in J. Sambrook *et* *al., Molecular Cloning,* Chapter 1, (1989).

Once isolated from the bacterial cells, the plasmid DNA served as a source for the DNA encoding the human HSGluR1 receptor protein. One convenient method to remove the receptor encoding DNA from plasmid pRS103 was to digest the plasmid with restriction enzymes EcoRI and Kpn I. These enzymes cut the plasmid at unique sites to produce a DNA fragment of approximately 4.2 kb containing the entire coding sequence of the human HSGluR1 receptor.

### Example 2

### In vitro transcription of RNA usina pRS103 as a DNA template

RNA transcripts encoding the HSGluR1 receptor were produced by enzymatic transcription from pRS103 using an RNA polymerase which recognizes the transcription promoter contained in the plasmid adjacent to the amino terminal coding end of the receptor subunit cDNA. Plasmid pRS103 was treated with the restriction enzyme SalI which made a single cut distal to the 3' end of the cDNA insert in the circular DNA and converted the plasmid DNA into a linear form. This DNA was then incubated with T7 RNA polymerase in the presence of GpppG cap nucleotide, rATP, rCTP, rUTP and rGTP. The synthetic RNA transcript obtained was purified by passage over a Sephadex G-50 column. For a detailed description of *in vitro* RNA synthesis using bacteriophage RNA polymerase such as T7, see P. A. Krieg and D. A. Melton, Vol 155, *Methods in Enzymology,* Ch. 25, 1987.

### Example 3

### Functional Expression of Human HSGluR1 Receptor in Xenopus Oocytes.

Oocytes suitable for injection were obtained from the adult female *Xenopus laevis* using procedures described in C. J. Marcus-Sekura and M. J. M. Hitchcock, *Methods in Enzymology,* Vol. 152 (1987). After treatment with collagenase type 1a (Sigma) at a concentration of 2 mg/ml, the defolliculated oocytes were injected essentially as described by M. J. M. Hitchcock *et al.,* *Methods in Enzymology*, Vol. 152 Chapter 28, 1987). Subsequently, 5 ng of RNA transcript in a total volume of 50 nl, prepared as described in Example 2, were injected into each oocyte and they were then incubated in Barth's saline solution at 18°C until needed for electrophysiological measurements.

In order to detect the presence of HSGluR1 receptor, the ability of the receptor to assemble into functional ion channels was determined by voltage recording of electrical current flowing across the oocyte membrane in response to glutamate agonists. Individual oocytes were placed in a diffusion chamber (0.5 ml vol.) through which solutions were perfused rapidly. Drugs (agonists and antagonists) were applied to the oocytes by adding them to the perfusing solutions and subsequently washing them out with control solution. The control solution contained 96 nM NaCl, 2mM KCl, 1.8 nM CaCl2, 1 mM MgCl2, and 5 mM HEPES buffer, pH 7.6. After insertion of electrodes into the oocytes, voltage recordings were made using the bridge circuit of an Axoclamp 1A voltage-clamp unit. Microelectrodes were filled with 3 M CsCl. Electrophysiological recordings of the oocytes clamped at -70 mV were made at room temperature (20-25°C), 3 days or more after injection of RNA into the oocytes. In response to perfusion of the cells with 100 µM kainic acid, an inward current across the oocyte membrane of 10-30 nanoamperes was observed. For a detailed discussion of the electrophysiology of Xenopus oocytes see N. Dascal, *22 CRC Critical Reviews in* *Biochemistry,* 317 (1987). As those skilled in the art appreciate these results are indicative of a glutamate receptor.

### Example 4

### Growth of E. coli/pRS113

A lyophilized culture of *E. coli* containing plasmid pRS113 can be obtained from the American Type Culture Collection, Rockville, Maryland 20852, and inoculated into a suitable broth for the growth of *E.* *coli* using standard microbiological procedures.

The contents of a lyophil vial containing *E.coli*/pRS113 were transferred into 100 ml of sterile YT (tryptone-yeast extract) broth containing 100 µg/ml ampicillin in a one liter fermentation flask and shaken at 37°C on an orbital shaker at 250-300 rpm. After the optical density (OD, measured at 600 millimicrons) had reached approximately 1-2 OD, the bacterial cells were recovered and used for the isolation of plasmid pRS113 according to the procedures detailed in J. Sambrook *et* *al., Molecular Cloning,* Chapter 1, (1989).

Once isolated from the bacterial cells, the plasmid DNA served as a source for the DNA encoding the human HSGluR2 receptor protein. One convenient method to remove the receptor encoding DNA from plasmid pRS113 was to digest the plasmid with restriction enzymes AlwnI and SalI. These enzymes cut the plasmid at unique sites to produce a DNA fragment of approximately 2.9kb containing the entire coding sequence of the human HSGluR2 receptor.

### Example 5

### In vitro transcription of RNA using pRS113 as a DNA template

RNA transcripts encoding the HSGluR2 receptor were produced by enzymatic transcription from pRS113 using an RNA polymerase which recognizes the transcription promoter contained in the plasmid adjacent to the amino terminal coding end of the receptor subunit cDNA. Plasmid pRS113 was treated with the restriction enzyme SalI which made a single cut distal to the 3' end of the cDNA insert in the circular DNA and converted the plasmid DNA into a linear form. This DNA was then incubated with T7 RNA poly-merase in the presence of GpppG cap nucleotide, rATP, rCTP, rUTP and rGTP. The synthetic RNA transcript obtained was purified by passage over a Sephadex G-50 column. For a detailed description of *in vitro* RNA synthesis using bacteriophage RNA polymerase such as T7, see P. A. Krieg and D. A. Melton, Vol 155, *Methods in Enzymology,* Ch. 25, 1987.

### Example 6

### Functional Expression of Human HSGluR2 Receptor in Xenopus Oocytes.

Oocytes suitable for injection were obtained from the adult female *Xenopus laevis* using procedures described in C. J. Marcus-Sekura and M. J. M. Hitchcock, *Methods in Enzymology,* Vol. 152 (1987). After treatment with collagenase type la (Sigma) at a concentration of 2 mg/ml, the defolliculated oocytes were injected essentially as described by M. J. M. Hitchcock *et al., Methods in Enzymology,* Vol. 152 Chapter 28, 1987). Subsequently, 5-10 ng of RNA transcript in a total volume of 50 nl, prepared as described in Example 2, were injected into each oocyte and they were then incubated in Barth's saline solution at 18°C until needed for electrophysiological measurements.

In order to detect the presence of HSGluR2 receptor, the ability of the receptor to assemble into functional ion channels was determined by voltage recording of electrical current flowing across the oocyte membrane in response to glutamate agonists. Individual oocytes were placed in a diffusion chamber (0.5 ml vol.) through which solutions were perfused rapidly. Drugs (agonists and antagonists) were applied to the oocytes by adding them to the perfusing solutions and subsequently washing them out with control solution. The control solution contained 96 mM NaCl, 2mM KCl, 1.8 mM CaCl2, 1 mM MgCl2, and 5 mM HEPES buffer, pH 7.6. After insertion of electrodes into the oocytes, voltage recordings were made using the bridge circuit of an Axoclamp 1A voltage-clamp unit. Microelectrodes were filled with 3 M CsCl. Electrophysiological recordings of the oocytes clamped at -70 mV were made at room temperature (20-25°C), 3 days or more after injection of RNA into the oocytes. In response to perfusion of the cells with 1 mM L-glutamate, inward currents across the oocyte membrane of 3 to 5 nanoamperes was observed. When RNA transcripts (5 ng each) which encoded both HSGluR2 and HSGluR1 were coinjected, currents of approximately 70 nanoamperes were observed in response to perfusion with 40 µM kainic acid. For a detailed discussion of the electrophysiology of Xenopus oocytes see Dascal N., 22 *CRC Critical Reviews in Biochemistry,* 317 (1987). As those skilled in the art appreciate these results are indicative of a glutamate receptor.

## Claims

1. A human glutamate receptor protein which comprises the amino acid sequence of SEQ ID NO:1, SEQ ID NO:3, or functional equivalents thereof.

2. The human glutamate receptor protein of Claim 1 which is SEQ ID NO:1.

3. The human glutamate receptor protein of Claim 1 which is SEQ ID NO:3.

4. A nucleic acid compound which comprises an isolated nucleic acid sequence which encodes all or part of a protein of Claim 1.

5. The nucleic acid compound of Claim 4 which is SEQ ID NO:2 or a functional equivalent thereof.

6. The nucleic acid compound of Claim 4 which is SEQ ID NO:4 or a functional equivalent thereof.

7. A nucleic acid vector which comprises the nucleic acid compound of Claim 4, 5, or 6.

8. A host cell comprising a nucleic acid vector of Claim 7.

9. The host cell of Claim 8 which is *E. coli*/pRS103 or *E. coli*/pRS113.

10. A method for expressing a nucleic acid compound which encodes SEQ ID NO:1, SEQ ID NO:3, or a functional equivalent thereof in a host cell transfected with said nucleic acid compound, said method comprising culturing said transfected host cell of Claim 8 under conditions suitable for expression of said necleic acid compound.

11. A method for determining whether a substance interacts with or affects HSGluR1 or HSGluR2, said method comprising introducing said substance and HSGluR1 and/or HSGluR2 into an acceptable medium, and monitoring the interaction by physically detectable means thereby identifying those substances which interact with or affect HSGluR1 and/or HSGluR2.

12. A method for constructing a host cell capable of expressing the protein of Claim 1, said method comprising transforming a host cell with a recombinant nucleic acid vector that comprises the nucleic acid compound of Claim 4, and culturing said host cell under conditions suitable for the expression of said nucleic acid compounds.


SEQUENCE LISTING

(1) GENERAL INFORMATION:

(i) APPLICANT: ELI LILLY AND COMPANY

(ii) TITLE OF INVENTION: HUMAN GLUTAMATE RECEPTOR PROPTEINS AND
RELATED DNA COMPOUNDS

(iii) NUMBER OF SEQUENCES: 10

(iv) CORRESPONDENCE ADDRESS:
(A) ADDRESSEE: Erl Wood Manor
(B) STREET: -
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Diskette, 3.5 inch
(B) COMPUTER: Macintosh
(C) OPERATING SYSTEM: Macintosh 7.0
(D) SOFTWARE: Microsoft Word

(2) INFORMATION FOR SEQ ID NO:1:

(i) SEQUENCE CHARACTERISTICS:
(A) LENGTH: 906 amino acids
(B) TYPE: amino acid
(D) TOPOLOGY: linear

(ii) MOLECULE TYPE: protein

(xi) SEQUENCE DESCRIPTION: SEQ ID NO:1:

Met Gln His Ile Phe Ala Phe Phe Cys Thr Gly Phe Leu Gly Ala Val
1               5                   10                  15

Val Gly Ala Asn Phe Pro Asn Asn Ile Gln Ile Gly Gly Leu Phe Pro
            20                  25                  30

Asn Gln Gln Ser Gln Glu His Ala Ala Phe Arg Phe Ala Leu Ser Gln
        35                  40                  45

Leu Thr Glu Pro Pro Lys Leu Leu Pro Gln Ile Asp Ile Val Asn Ile
    50                  55                  60

Ser Asp Ser Phe Glu Met Thr Tyr Arg Phe Cys Ser Gln Phe Ser Lys
65                  70                  75                  80

Gly Val Tyr Ala Ile Phe Gly Phe Tyr Glu Arg Arg Thr Val Asn Met
                85                  90                  95

Leu Thr Ser Phe Cys Gly Ala Leu His Val Cys Phe Ile Thr Pro Ser
            100                 105                 110

Phe Pro Val Asp Thr Ser Asn Gln Phe Val Leu Gln Leu Arg Pro Glu
        115                 120                 125

Leu Gln Asp Ala Leu Ile Ser Ile Ile Asp His Tyr Lys Trp Gln Lys
    130                 135                 140

Phe Val Tyr Ile Tyr Asp Ala Asp Arg Gly Leu Ser Val Leu Gln Lys
145                 150                 155                 160

Val Leu Asp Thr Ala Ala Glu Lys Asn Trp Gln Val Thr Ala Val Asn
                165                 170                 175

Ile Leu Thr Thr Thr Glu Glu Gly Tyr Arg Met Leu Phe Gln Asp Leu
            180                 185                 190

Glu Lys Lys Lys Glu Arg Leu Val Val Val Asp Cys Glu Ser Glu Arg
        195                 200                 205

Leu Asn Ala Ile Leu Gly Gln Ile Ile Lys Leu Glu Lys Asn Gly Ile
    210                 215                 220

Gly Tyr His Tyr Ile Leu Ala Asn Leu Gly Phe Met Asp Ile Asp Leu
225                 230                 235                 240

Asn Lys Phe Lys Glu Ser Gly Ala Asn Val Thr Gly Phe Gln Leu Val
                245                 250                 255

Asn Tyr Thr Asp Thr Ile Pro Ala Lys Ile Met Gln Gln Trp Lys Asn
            260                 265                 270

Ser Asp Ala Arg Asp His Thr Arg Val Asp Trp Lys Arg Pro Lys Tyr
        275                 280                 285

Thr Ser Ala Leu Thr Tyr Asp Gly Val Lys Val Met Ala Glu Ala Phe
    290                 295                 300

Gln Ser Leu Arg Arg Gln Arg Ile Asp Ile Ser Arg Arg Gly Asn Ala
305                 310                 315                 320

Gly Asp Cys Leu Ala Asn Pro Ala Val Pro Trp Gly Gln Gly Ile Asp
                325                 330                 335

Ile Gln Arg Ala Leu Gln Gln Val Arg Phe Glu Gly Leu Thr Gly Asn
            340                 345                 350

Val Gln Phe Asn Glu Lys Gly Arg Arg Thr Asn Tyr Thr Leu His Val
        355                 360                 365

Ile Glu Met Lys His Asp Gly Ile Arg Lys Ile Gly Tyr Trp Asn Glu
    370                 375                 380

Asp Asp Lys Phe Val Pro Ala Ala Thr Asp Ala Gln Ala Gly Gly Asp
385                 390                 395                 400

Asn Ser Ser Val Gln Asn Arg Thr Tyr Ile Val Thr Thr Ile Leu Glu
                405                 410                 415

Asp Pro Tyr Val Met Leu Lys Lys Asn Ala Asn Gln Phe Glu Gly Asn
            420                 425                 430

Asp Arg Tyr Glu Gly Tyr Cys Val Glu Leu Ala Ala Glu Ile Ala Lys
        435                 440                 445

His Val Gly Tyr Ser Tyr Arg Leu Glu Ile Val Ser Asp Gly Lys Tyr
    450                 455                 460

Gly Ala Arg Asp Pro Asp Thr Lys Ala Trp Asn Gly Met Val Gly Glu
465                 470                 475                 480

Leu Val Tyr Gly Arg Ala Asp Val Ala Val Ala Pro Leu Thr Ile Thr
                485                 490                 495

Leu Val Arg Glu Glu Val Ile Asp Phe Ser Lys Pro Phe Met Ser Leu
            500                 505                 510

Gly Ile Ser Ile Met Ile Lys Lys Pro Gln Lys Ser Lys Pro Gly Val
        515                 520                 525

Phe Ser Phe Leu Asp Pro Leu Ala Tyr Glu Ile Trp Met Cys Ile Val
    530                 535                 540

Phe Ala Tyr Ile Gly Val Ser Val Val Leu Phe Leu Val Ser Arg Phe
545                 550                 555                 560

Ser Pro Tyr Glu Trp His Ser Glu Glu Phe Glu Glu Gly Arg Asp Gln
                565                 570                 575

Thr Thr Ser Asp Gln Ser Asn Glu Phe Gly Ile Phe Asn Ser Leu Trp
            580                 585                 590

Phe Ser Leu Gly Ala Phe Met Gln Gln Gly Cys Asp Ile Ser Pro Arg
        595                 600                 605

Ser Leu Ser Gly Arg Ile Val Gly Gly Val Trp Trp Phe Phe Thr Leu
    610                 615                 620

Ile Ile Ile Ser Ser Tyr Thr Ala Asn Leu Ala Ala Phe Leu Thr Val
625                 630                 635                 640

Glu Arg Met Val Ser Pro Ile Glu Ser Ala Glu Asp Leu Ala Lys Gln
                645                 650                 655

Thr Glu Ile Ala Tyr Gly Thr Leu Glu Ala Gly Ser Thr Lys Glu Phe
            660                 665                 670

Phe Arg Arg Ser Lys Ile Ala Val Phe Glu Lys Met Trp Thr Tyr Met
        675                 680                 685

Lys Ser Ala Glu Pro Ser Val Phe Val Arg Thr Thr Glu Glu Gly Met
    690                 695                 700

Ile Arg Val Arg Lys Ser Lys Gly Lys Tyr Ala Tyr Leu Leu Glu Ser
705                 710                 715                 720

Thr Met Asn Glu Tyr Ile Glu Gln Arg Lys Pro Cys Asp Thr Met Lys
                725                 730                 735

Val Gly Gly Asn Leu Asp Ser Lys Gly Tyr Gly Ile Ala Thr Pro Lys
            740                 745                 750

Gly Ser Ala Leu Arg Asn Pro Val Asn Leu Ala Val Leu Lys Leu Asn
        755                 760                 765

Glu Gln Gly Leu Leu Asp Lys Leu Lys Asn Lys Trp Trp Tyr Asp Lys
    770                 775                 780

Gly Glu Cys Gly Ser Gly Gly Gly Asp Ser Lys Asp Lys Thr Ser Ala
785                 790                 795                 800

Leu Ser Leu Ser Asn Val Ala Gly Val Phe Tyr Ile Leu Ile Gly Gly
                805                 810                 815

Leu Gly Leu Ala Met Leu Val Ala Leu Ile Glu Phe Cys Tyr Lys Ser
            820                 825                 830

Arg Ser Glu Ser Lys Arg Met Lys Gly Phe Cys Leu Ile Pro Gln Gln
        835                 840                 845

Ser Ile Asn Glu Ala Ile Arg Thr Ser Thr Leu Pro Arg Asn Ser Gly
    850                 855                 860

Ala Gly Ala Ser Ser Gly Gly Ser Gly Glu Asn Gly Arg Val Val Ser
865                 870                 875                 880

His Asp Phe Pro Lys Ser Met Gln Ser Ile Pro Cys Met Ser His Ser
                885                 890                 895

Ser Gly Met Pro Leu Gly Ala Thr Gly Leu
            900                 905

(3) INFORMATION FOR SEQ ID NO:2:

(i) SEQUENCE CHARACTERISTICS:
(A) LENGTH: 2718 base pairs
(B) TYPE: nucleic acid
(C) STRANDEDNESS: both
(D) TOPOLOGY: linear

(ii) MOLECULE TYPE: cDNA

(ix) FEATURE:
(A) NAME/KEY: CDS
(B) LOCATION: 1..2718

(xi) SEQUENCE DESCRIPTION: SEQ ID NO:2:

ATG CAG CAC ATT TTT GCC TTC TTC TGC ACC GGT TTC CTA GGC GCG GTA      48
Met Gln His Ile Phe Ala Phe Phe Cys Thr Gly Phe Leu Gly Ala Val
1               5                   10                  15

GTA GGT GCC AAT TTC CCC AAC AAT ATC CAG ATC GGG GGA TTA TTT CCA      96
Val Gly Ala Asn Phe Pro Asn Asn Ile Gln Ile Gly Gly Leu Phe Pro
            20                  25                  30

AAC CAG CAG TCA CAG GAA CAT GCT GCT TTT AGA TTT GCT TTG TCG CAA      144
Asn Gln Gln Ser Gln Glu His Ala Ala Phe Arg Phe Ala Leu Ser Gln
        35                  40                  45

CTC ACA GAG CCC CCG AAG CTG CTC CCC CAG ATT GAT ATT GTG AAC ATC      192
Leu Thr Glu Pro Pro Lys Leu Leu Pro Gln Ile Asp Ile Val Asn Ile
    50                  55                  60

AGC GAC AGC TTT GAG ATG ACC TAT AGA TTC TGT TCC CAG TTC TCC AAA      240
Ser Asp Ser Phe Glu Met Thr Tyr Arg Phe Cys Ser Gln Phe Ser Lys
65                  70                  75                  80

GGA GTC TAT GCC ATC TTT GGG TTT TAT GAA CGT AGG ACT GTC AAC ATG      288
Gly Val Tyr Ala Ile Phe Gly Phe Tyr Glu Arg Arg Thr Val Asn Met
                85                  90                  95

CTG ACC TCC TTT TGT GGG GCC CTC CAC GTC TGC TTC ATT ACG CCG AGC      336
Leu Thr Ser Phe Cys Gly Ala Leu His Val Cys Phe Ile Thr Pro Ser
            100                 105                 110

TTT CCC GTT GAT ACA TCC AAT CAG TTT GTC CTT CAG CTG CGC CCT GAA 384
Phe Pro Val Asp Thr Ser Asn Gln Phe Val Leu Gln Leu Arg Pro Glu
        115                 120                 125

CTG CAG GAT GCC CTC ATC AGC ATC ATT GAC CAT TAC AAG TGG CAG AAA 432
Leu Gln Asp Ala Leu Ile Ser Ile Ile Asp His Tyr Lys Trp Gln Lys
    130                 135                 140

TTT GTC TAC ATT TAT GAT GCC GAC CGG GGC TTA TCC GTC CTG CAG AAA 480
Phe Val Tyr Ile Tyr Asp Ala Asp Arg Gly Leu Ser Val Leu Gln Lys
145                 150                 155                 160

GTC CTG GAT ACA GCT GCT GAG AAG AAC TGG CAG GTG ACA GCA GTC AAC 528
Val Leu Asp Thr Ala Ala Glu Lys Asn Trp Gln Val Thr Ala Val Asn
                165                 170                 175

ATT TTG ACA ACC ACA GAG GAG GGA TAC CGG ATG CTC TTT CAG GAC CTG 576
Ile Leu Thr Thr Thr Glu Glu Gly Tyr Arg Met Leu Phe Gln Asp Leu
            180                 185                 190

GAG AAG AAA AAG GAG CGG CTG GTG GTG GTG GAC TGT GAA TCA GAA CGC 624
Glu Lys Lys Lys Glu Arg Leu Val Val Val Asp Cys Glu Ser Glu Arg
        195                 200                 205

CTC AAT GCT ATC TTG GGC CAG ATT ATA AAG CTA GAG AAG AAT GGC ATC 672
Leu Asn Ala Ile Leu Gly Gln Ile Ile Lys Leu Glu Lys Asn Gly Ile
    210                 215                 220

GGC TAC CAC TAC ATT CTT GCA AAT CTG GGC TTC ATG GAC ATT GAC TTA 720
Gly Tyr His Tyr Ile Leu Ala Asn Leu Gly Phe Met Asp Ile Asp Leu
225                 230                 235                 240

AAC AAA TTC AAG GAG AGT GGC GCC AAT GTG ACA GGT TTC CAG CTG GTG 768
Asn Lys Phe Lys Glu Ser Gly Ala Asn Val Thr Gly Phe Gln Leu Val
                245                 250                 255

AAC TAC ACA GAC ACT ATT CCG GCC AAG ATC ATG CAG CAG TGG AAG AAT 816
Asn Tyr Thr Asp Thr Ile Pro Ala Lys Ile Met Gln Gln Trp Lys Asn
            260                 265                 270

AGT GAT GCT CGA GAC CAC ACA CGG GTG GAC TGG AAG AGA CCC AAG TAC 864
Ser Asp Ala Arg Asp His Thr Arg Val Asp Trp Lys Arg Pro Lys Tyr
        275                 280                 285

ACC TCT GCG CTC ACC TAC GAT GGG GTG AAG GTG ATG GCT GAG GCT TTC 912
Thr Ser Ala Leu Thr Tyr Asp Gly Val Lys Val Met Ala Glu Ala Phe
    290                 295                 300

CAG AGC CTG CGG AGG CAG AGA ATT GAT ATA TCT CGC CGG GGG AAT GCT 960
Gln Ser Leu Arg Arg Gln Arg Ile Asp Ile Ser Arg Arg Gly Asn Ala
305                 310                 315                 320

GGG GAT TGT CTG GCT AAC CCA GCT GTT CCC TGG GGC CAA GGG ATC GAC 1008
Gly Asp Cys Leu Ala Asn Pro Ala Val Pro Trp Gly Gln Gly Ile Asp

                325                 330                 335

ATC CAG AGA GCT CTG CAG CAG GTG CGA TTT GAA GGT TTA ACA GGA AAC 1056
Ile Gln Arg Ala Leu Gln Gln Val Arg Phe Glu Gly Leu Thr Gly Asn
            340                 345                 350

GTG CAG TTT AAT GAG AAA GGA CGC CGG ACC AAC TAC ACG CTC CAC GTG 1104
Val Gln Phe Asn Glu Lys Gly Arg Arg Thr Asn Tyr Thr Leu His Val
        355                 360                 365

ATT GAA ATG AAA CAT GAC GGC ATC CGA AAG ATT GGT TAC TGG AAT GAA 1152
Ile Glu Met Lys His Asp Gly Ile Arg Lys Ile Gly Tyr Trp Asn Glu
    370                 375                 380

GAT GAT AAG TTT GTC CCT GCA GCC ACC GAT GCC CAA GCT GGG GGC GAT 1200
Asp Asp Lys Phe Val Pro Ala Ala Thr Asp Ala Gln Ala Gly Gly Asp
385                 390                 395                 400

AAT TCA AGT GTT CAG AAC AGA ACA TAC ATC GTC ACA ACA ATC CTA GAA 1248
Asn Ser Ser Val Gln Asn Arg Thr Tyr Ile Val Thr Thr Ile Leu Glu
                405                 410                 415

GAT CCT TAT GTG ATG CTC AAG AAG AAC GCC AAT CAG TTT GAG GGC AAT 1296
Asp Pro Tyr Val Met Leu Lys Lys Asn Ala Asn Gln Phe Glu Gly Asn
            420                 425                 430

GAC CGT TAC GAG GGC TAC TGT GTA GAG CTG GCG GCA GAG ATT GCC AAG 1344
Asp Arg Tyr Glu Gly Tyr Cys Val Glu Leu Ala Ala Glu Ile Ala Lys
        435                 440                 445

CAC GTG GGC TAC TCC TAC CGT CTG GAG ATT GTC AGT GAT GGA AAA TAC 1392
His Val Gly Tyr Ser Tyr Arg Leu Glu Ile Val Ser Asp Gly Lys Tyr
    450                 455                 460

GGA GCC CGA GAC CCT GAC ACG AAG GCC TGG AAT GGC ATG GTG GGA GAG 1440
Gly Ala Arg Asp Pro Asp Thr Lys Ala Trp Asn Gly Met Val Gly Glu
465                 470                 475                 480

CTG GTC TAT GGA AGA GCA GAT GTG GCT GTG GCT CCC TTA ACT ATC ACT 1488
Leu Val Tyr Gly Arg Ala Asp Val Ala Val Ala Pro Leu Thr Ile Thr
                485                 490                 495

TTG GTC CGG GAA GAA GTT ATA GAT TTC TCC AAA CCA TTT ATG AGT TTG 1536
Leu Val Arg Glu Glu Val Ile Asp Phe Ser Lys Pro Phe Met Ser Leu
            500                 505                 510

GGG ATC TCC ATC ATG ATT AAA AAA CCA CAG AAA TCC AAG CCG GGT GTC 1584
Gly Ile Ser Ile Met Ile Lys Lys Pro Gln Lys Ser Lys Pro Gly Val
        515                 520                 525

TTC TCC TTC CTT GAT CCT TTG GCT TAT GAG ATT TGG ATG TGC ATT GTT 1632
Phe Ser Phe Leu Asp Pro Leu Ala Tyr Glu Ile Trp Met Cys Ile Val
    530                 535                 540

TTT GCC TAC ATT GGA GTG AGT GTT GTC CTC TTC CTG GTC AGC CGC TTC 1680
Phe Ala Tyr Ile Gly Val Ser Val Val Leu Phe Leu Val Ser Arg Phe
545                 550                 555                 560

AGT CCC TAT GAA TGG CAC AGT GAA GAG TTT GAG GAA GGA CGG GAC CAG 1728
Ser Pro Tyr Glu Trp His Ser Glu Glu Phe Glu Glu Gly Arg Asp Gln
                565                 570                 575

ACA ACC AGT GAC CAG TCC AAT GAG TTT GGG ATA TTC AAC AGT TTG TGG 1776
Thr Thr Ser Asp Gln Ser Asn Glu Phe Gly Ile Phe Asn Ser Leu Trp
            580                 585                 590

TTC TCC CTG GGA GCC TTC ATG CAG CAA GGA TGT GAC ATT TCT CCC AGG 1824
Phe Ser Leu Gly Ala Phe Met Gln Gln Gly Cys Asp Ile Ser Pro Arg
        595                 600                 605

TCC CTG TCT GGT CGC ATC GTT GGT GGC GTC TGG TGG TTC TTC ACC TTA 1872
Ser Leu Ser Gly Arg Ile Val Gly Gly Val Trp Trp Phe Phe Thr Leu
    610                 615                 620

ATC ATC ATC TCC TCA TAT ACA GCC AAT CTG GCC GCC TTC CTG ACC GTG 1920
Ile Ile Ile Ser Ser Tyr Thr Ala Asn Leu Ala Ala Phe Leu Thr Val
625                 630                 635                 640

GAG AGG ATG GTG TCT CCC ATT GAG AGT GCA GAG GAC CTA GCG AAG CAG 1968
Glu Arg Met Val Ser Pro Ile Glu Ser Ala Glu Asp Leu Ala Lys Gln
                645                 650                 655

ACA GAA ATT GCC TAC GGG ACG CTG GAA GCA GGA TCT ACT AAG GAG TTC 2016
Thr Glu Ile Ala Tyr Gly Thr Leu Glu Ala Gly Ser Thr Lys Glu Phe
            660                 665                 670

TTC AGG AGG TCT AAA ATT GCT GTG TTT GAG AAG ATG TGG ACA TAC ATG 2064
Phe Arg Arg Ser Lys Ile Ala Val Phe Glu Lys Met Trp Thr Tyr Met
        675                 680                 685

AAG TCA GCA GAG CCA TCA GTT TTT GTG CGG ACC ACA GAG GAG GGG ATG 2112
Lys Ser Ala Glu Pro Ser Val Phe Val Arg Thr Thr Glu Glu Gly Met
    690                 695                 700

ATT CGA GTG AGG AAA TCC AAA GGC AAA TAT GCC TAC CTC CTG GAG TCC 2160
Ile Arg Val Arg Lys Ser Lys Gly Lys Tyr Ala Tyr Leu Leu Glu Ser
705                 710                 715                 720

ACC ATG AAT GAG TAC ATT GAG CAG CGG AAA CCC TGT GAC ACC ATG AAG 2208
Thr Met Asn Glu Tyr Ile Glu Gln Arg Lys Pro Cys Asp Thr Met Lys
                725                 730                 735

GTG GGA GGT AAC TTG GAT TCC AAA GGC TAT GGC ATT GCA ACA CCC AAG 2256
Val Gly Gly Asn Leu Asp Ser Lys Gly Tyr Gly Ile Ala Thr Pro Lys
            740                 745                 750

GGG TCT GCC CTG AGA AAT CCA GTA AAC CTG GCA GTG TTA AAA CTG AAC 2304
Gly Ser Ala Leu Arg Asn Pro Val Asn Leu Ala Val Leu Lys Leu Asn

        755                 760                 765

GAG CAG GGG CTT TTG GAC AAA TTG AAA AAC AAA TGG TGG TAC GAC AAG 2352
Glu Gln Gly Leu Leu Asp Lys Leu Lys Asn Lys Trp Trp Tyr Asp Lys
    770                 775                 780

GGC GAG TGC GGC AGC GGG GGA GGT GAT TCC AAG GAC AAG ACA AGC GCT 2400
Gly Glu Cys Gly Ser Gly Gly Gly Asp Ser Lys Asp Lys Thr Ser Ala
785                 790                 795                 800

CTG AGC CTC AGC AAT GTG GCA GGC GTG TTC TAC ATC CTG ATC GGA GGA 2448
Leu Ser Leu Ser Asn Val Ala Gly Val Phe Tyr Ile Leu Ile Gly Gly
                805                 810                 815

CTT GGA CTA GCC ATG CTG GTT GCC TTA ATC GAG TTC TGC TAC AAA TCC 2496
Leu Gly Leu Ala Met Leu Val Ala Leu Ile Glu Phe Cys Tyr Lys Ser
            820                 825                 830

CGT AGT GAA TCC AAG CGG ATG AAG GGT TTT TGT TTG ATC CCA CAG CAA 2544
Arg Ser Glu Ser Lys Arg Met Lys Gly Phe Cys Leu Ile Pro Gln Gln
        835                 840                 845

TCC ATC AAC GAA GCC ATA CGG ACA TCG ACC CTC CCC CGC AAC AGC GGG 2592
Ser Ile Asn Glu Ala Ile Arg Thr Ser Thr Leu Pro Arg Asn Ser Gly
    850                 855                 860

GCA GGA GCC AGC AGC GGC GGC AGT GGA GAG AAT GGT CGG GTG GTC AGC 2640
Ala Gly Ala Ser Ser Gly Gly Ser Gly Glu Asn Gly Arg Val Val Ser
865                 870                 875                 880

CAT GAC TTC CCC AAG TCC ATG CAA TCG ATT CCT TGC ATG AGC CAC AGT 2688
His Asp Phe Pro Lys Ser Met Gln Ser Ile Pro Cys Met Ser His Ser
                885                 890                 895

TCA GGG ATG CCC TTG GGA GCC ACG GGA TTG 2718
Ser Gly Met Pro Leu Gly Ala Thr Gly Leu
            900                 905

(4) INFORMATION FOR SEQ ID NO:3:

(i) SEQUENCE CHARACTERISTICS:
(A) LENGTH: 883 amino acids
(B) TYPE: amino acid
(D) TOPOLOGY: linear

(ii) MOLECULE TYPE: protein

(xi) SEQUENCE DESCRIPTION: SEQ ID NO:3:

Met Gln Lys Ile Met His Ile Ser Val Leu Leu Ser Pro Val Leu Trp
1               5                   10                  15

Gly Leu Ile Phe Gly Val Ser Ser Asn Ser Ile Gln Ile Gly Gly Leu

20                  25                  30

Phe Pro Arg Gly Ala Asp Gln Glu Tyr Ser Ala Phe Arg Val Gly Met
        35                  40                  45

Val Gln Phe Ser Thr Ser Glu Phe Arg Leu Thr Pro His Ile Asp Asn
    50                  55                  60

Leu Glu Val Ala Asn Ser Phe Ala Val Thr Asn Ala Phe Cys Ser Gln
65                  70                  75                  80

Phe Ser Arg Gly Val Tyr Ala Ile Phe Gly Phe Tyr Asp Lys Lys Ser
                85                  90                  95

Val Asn Thr Ile Thr Ser Phe Cys Gly Thr Leu His Val Ser Phe Ile
            100                 105                 110

Thr Pro Ser Phe Pro Thr Asp Gly Thr His Pro Phe Val Ile Gln Met
        115                 120                 125

Arg Pro Asp Leu Lys Gly Ala Leu Leu Ser Leu Ile Glu Tyr Tyr Gln
    130                 135                 140

Trp Asp Lys Phe Ala Tyr Leu Tyr Asp Ser Asp Arg Gly Leu Ser Thr
145                 150                 155                 160

Leu Gln Ala Val Leu Asp Ser Ala Ala Glu Lys Lys Trp Gln Val Thr
                165                 170                 175

Ala Ile Asn Val Gly Asn Ile Asn Asn Asp Lys Lys Asp Glu Met Tyr
            180                 185                 190

Arg Ser Leu Phe Gln Asp Leu Glu Leu Lys Lys Glu Arg Arg Val Ile
        195                 200                 205

Leu Asp Cys Glu Arg Asp Lys Val Asn Asp Ile Val Asp Gln Val Ile
    210                 215                 220

Thr Ile Gly Lys His Val Lys Gly Tyr His Tyr Ile Ile Ala Asn Leu
225                 230                 235                 240

Gly Phe Thr Asp Gly Asp Leu Leu Lys Ile Gln Phe Gly Gly Ala Asn
                245                 250                 255

Val Ser Gly Phe Gln Ile Val Asp Tyr Asp Asp Ser Leu Val Ser Lys
            260                 265                 270

Phe Ile Glu Arg Trp Ser Thr Leu Glu Glu Lys Glu Tyr Pro Gly Ala
        275                 280                 285

His Thr Thr Thr Ile Lys Tyr Thr Ser Ala Leu Thr Tyr Asp Ala Val
    290                 295                 300

Gln Val Met Thr Glu Ala Phe Arg Asn Leu Arg Lys Gln Arg Ile Glu

305                 310                 315                 320

Ile Ser Arg Arg Gly Asn Ala Gly Asp Cys Leu Ala Asn Pro Ala Val
                325                 330                 335

Pro Trp Gly Gln Gly Val Glu Ile Glu Arg Ala Leu Lys Gln Val Gln
            340                 345                 350

Val Glu Gly Leu Ser Gly Asn Ile Lys Phe Asp Gln Asn Gly Lys Arg
        355                 360                 365

Ile Asn Tyr Thr Ile Asn Ile Met Glu Leu Lys Thr Asn Gly Pro Arg
    370                 375                 380

Lys Ile Gly Tyr Trp Ser Glu Val Asp Lys Met Val Val Thr Leu Thr
385                 390                 395                 400

Glu Leu Pro Ser Gly Asn Asp Thr Ser Gly Leu Glu Asn Lys Thr Val
                405                 410                 415

Val Val Thr Thr Ile Leu Glu Ser Pro Tyr Val Met Met Lys Lys Asn
            420                 425                 430

His Glu Met Leu Glu Gly Asn Glu Arg Tyr Glu Gly Tyr Cys Val Asp
        435                 440                 445

Leu Ala Ala Glu Ile Ala Lys His Cys Gly Phe Lys Tyr Lys Leu Thr
    450                 455                 460

Ile Val Gly Asp Gly Lys Tyr Gly Ala Arg Asp Ala Asp Thr Lys Ile
465                 470                 475                 480

Trp Asn Gly Met Val Gly Glu Leu Val Tyr Gly Lys Ala Asp Ile Ala
                485                 490                 495

Ile Ala Pro Leu Thr Ile Thr Leu Val Arg Glu Glu Val Ile Asp Phe
            500                 505                 510

Ser Lys Pro Phe Met Ser Leu Gly Ile Ser Ile Met Ile Lys Lys Pro
        515                 520                 525

Gln Lys Ser Lys Pro Gly Val Phe Ser Phe Leu Asp Pro Leu Ala Tyr
    530                 535                 540

Glu Ile Trp Met Cys Ile Val Phe Ala Tyr Ile Gly Val Ser Val Val
545                 550                 555                 560

Leu Phe Leu Val Ser Arg Phe Ser Pro Tyr Glu Trp His Thr Glu Glu
                565                 570                 575

Phe Glu Asp Gly Arg Glu Thr Gln Ser Ser Glu Ser Thr Asn Glu Phe
            580                 585                 590

Gly Ile Phe Asn Ser Leu Trp Phe Ser Leu Gly Ala Phe Met Arg Gln

        595                 600                 605

Gly Cys Asp Ile Ser Pro Arg Ser Leu Ser Gly Arg Ile Val Gly Gly
    610                 615                 620

Val Trp Trp Phe Phe Thr Leu Ile Ile Ile Ser Ser Tyr Thr Ala Asn
625                 630                 635                 640

Leu Ala Ala Phe Leu Thr Val Glu Arg Met Val Ser Pro Ile Glu Ser
                645                 650                 655

Ala Glu Asp Leu Ser Lys Gln Thr Glu Ile Ala Tyr Gly Thr Leu Asp
            660                 665                 670

Ser Gly Ser Thr Lys Glu Phe Phe Arg Arg Ser Lys Ile Ala Val Phe
        675                 680                 685

Asp Lys Met Trp Thr Tyr Met Arg Ser Ala Glu Pro Ser Val Phe Val
    690                 695                 700

Arg Thr Thr Ala Glu Gly Val Ala Arg Val Arg Lys Ser Lys Gly Lys
705                 710                 715                 720

Tyr Ala Tyr Leu Leu Glu Ser Thr Met Asn Glu Tyr Ile Glu Gln Arg
                725                 730                 735

Lys Pro Cys Asp Thr Met Lys Val Gly Gly Asn Leu Asp Ser Lys Gly
            740                 745                 750

Tyr Gly Ile Ala Thr Pro Lys Gly Ser Ser Leu Arg Thr Pro Val Asn
        755                 760                 765

Leu Ala Val Leu Lys Leu Ser Glu Gln Gly Val Leu Asp Lys Leu Lys
    770                 775                 780

Asn Lys Trp Trp Tyr Asp Lys Gly Glu Cys Gly Ala Lys Asp Ser Gly
785                 790                 795                 800

Ser Lys Glu Lys Thr Ser Ala Leu Ser Leu Ser Asn Val Ala Gly Val
                805                 810                 815

Phe Tyr Ile Leu Val Gly Gly Leu Gly Leu Ala Met Leu Val Ala Leu
            820                 825                 830

Ile Glu Phe Cys Tyr Lys Ser Arg Ala Glu Ala Lys Arg Met Lys Val
        835                 840                 845

Ala Lys Asn Ala Gln Asn Ile Asn Pro Ser Ser Ser Gln Asn Ser Gln
    850                 855                 860

Asn Phe Ala Thr Tyr Lys Glu Gly Tyr Asn Val Tyr Gly Ile Glu Ser
865                 870                 875                 880

Val Lys Ile

(5) INFORMATION FOR SEQ ID NO:4:

(i) SEQUENCE CHARACTERISTICS:
(A) LENGTH: 2649 base pairs
(B) TYPE: nucleic acid
(C) STRANDEDNESS: both
(D) TOPOLOGY: linear

(ii) MOLECULE TYPE: cDNA

(xi) SEQUENCE DESCRIPTION: SEQ ID NO:4:

ATGCAAAAGA TTATGCATAT TTCTGTCCTC CTTTCTCCTG TTTTATGGGG ACTGATTTTT 60

GGTGTCTCTT CTAACAGCAT ACAGATAGGG GGGCTATTTC CTAGGGGCGC CGATCAAGAA 120

TACAGTGCAT TTCGAGTAGG GATGGTTCAG TTTTCCACTT CGGAGTTCAG ACTGACACCC 180

CACATCGACA ATTTGGAGGT GGCAAACAGC TTCGCAGTCA CTAATGCTTT CTGCTCCCAG 240

TTTTCGAGAG GAGTCTATGC TATTTTTGGA TTTTATGACA AGAAGTCTGT AAATACCATC 300

ACATCATTTT GCGGAACACT CCACGTCTCC TTCATCACTC CCAGCTTCCC AACAGATGGC 360

ACACATCCAT TTGTCATTCA GATGAGACCC GACCTCAAAG GAGCTCTCCT TAGCTTGATT 420

GAATACTATC AATGGGACAA GTTTGCATAC CTCTATGACA GTGACAGAGG CTTATCAACA 480

CTGCAAGCTG TGCTGGATTC TGCTGCTGAA AAGAAATGGC AAGTGACTGC TATCAATGTG 540

GGAAACATTA ACAATGACAA GAAAGATGAG ATGTACCGAT CACTTTTTCA AGATCTGGAG 600

TTAAAAAAGG AACGGCGTGT AATTCTGGAC TGTGAAAGGG ATAAAGTAAA CGACATTGTA 660

GACCAGGTTA TTACCATTGG AAAACATGTT AAAGGGTACC ACTACATCAT TGCAAATCTG 720

GGATTTACTG ATGGAGACCT ATTAAAAATC CAGTTTGGAG GTGCAAATGT CTCTGGATTT 780

CAGATAGTGG ACTATGATGA TTCGTTGGTA TCTAAATTTA TAGAAAGATG GTCAACACTG 840

GAAGAAAAAG AATACCCTGG AGCTCACACA ACAACAATTA AGTATACTTC TGCTCTGACC 900

TATGATGCCG TTCAAGTGAT GACTGAAGCC TTCCGCAACC TAAGGAAGCA AAGAATTGAA 960

ATCTCCCGAA GGGGGAATGC AGGAGACTGT CTGGCAAACC CAGCAGTGCC CTGGGGACAA 1020

GGTGTAGAAA TAGAAAGGGC CCTCAAACAG GTTCAGGTTG AAGGTCTCTC AGGAAATATA 1080

AAGTTTGACC AGAATGGAAA AAGAATAAAC TATACAATTA ACATCATGGA GCTCAAAACT 1140

AATGGGCCCC GGAAGATTGG CTACTGGAGT GAAGTGGACA AAATGGTTGT TACCCTTACT 1200

GAGCTCCCTT CTGGAAATGA CACCTCTGGG CTTGAGAATA AGACTGTTGT TGTCACCACA 1260

ATTTTGGAAT CTCCGTATGT TATGATGAAG AAAAATCATG AAATGCTTGA AGGCAATGAG 1320

CGCTATGAGG GCTACTGTGT TGACCTGGCT GCAGAAATCG CCAAACATTG TGGGTTCAAG 1380

TACAAGTTGA CAATTGTTGG TGATGGCAAG TATGGGGCCA GGGATGCAGA CACGAAAATT 1440

TGGAATGGGA TGGTTGGAGA ACTTGTATAT GGGAAAGCTG ATATTGCAAT TGCTCCATTA 1500

ACTATTACCC TTGTGAGAGA AGAGGTGATT GACTTCTCAA AGCCCTTCAT GAGCCTCGGG 1560

ATATCTATCA TGATCAAGAA GCCTCAGAAG TCCAAACCAG GAGTGTTTTC CTTTCTTGAT 1620

CCTTTAGCCT ATGAGATCTG GATGTGCATT GTTTTTGCCT ACATTGGGGT CAGTGTAGTT 1680

TTATTCCTGG TCAGCAGATT TAGCCCCTAC GAGTGGCACA CTGAGGAGTT TGAAGATGGA 1740

AGAGAAACAC AAAGTAGTGA ATCAACTAAT GAATTTGGGA TTTTTAATAG TCTCTGGTTT 1800

TCCTTGGGTG CCTTTATGCG GCAAGGATGC GATATTTCGC CAAGATCCCT CTCTGGGCGC 1860

ATTGTTGGAG GTGTGTGGTG GTTCTTTACC CTGATCATAA TCTCCTCCTA CACGGCTAAC 1920

TTAGCTGCCT TCCTGACTGT AGAGAGGATG GTGTCTCCCA TCGAAAGTGC TGAGGATCTT 1980

TCTAAGCAAA CAGAAATTGC TTATGGAACA TTAGACTCTG GCTCCACTAA AGAGTTTTTC 2040

AGGAGATCTA AAATTGCAGT GTTTGATAAA ATGTGGACCT ACATGCGGAG TGCGGAGCCC 2100

TCTGTGTTTG TGAGGACTAC GGCCGAAGGG GTGGCTAGAG TGCGGAAGTC CAAAGGGAAA 2160

TATGCCTACT TGTTGGAGTC CACGATGAAC GAGTACATTG AGCAAAGGAA GCCTTGCGAC 2220

ACCATGAAAG TTGGTGGAAA CCTGGATTCC AAAGGCTATG GCATCGCAAC ACCTAAAGGA 2280

TCCTCATTAA GAACCCCAGT AAATCTTGCA GTATTGAAAC TCAGTGAGCA AGGCGTCTTA 2340

GACAAGCTGA AAAACAAATG GTGGTACGAT AAAGGTGAAT GTGGAGCCAA GGACTCTGGA 2400

AGTAAGGAAA AGACCAGTGC CCTCAGTCTG AGCAACGTTG CTGGAGTATT CTACATCCTT 2460

GTCGGGGGCC TTGGTTTGGC AATGCTGGTG GCTTTGATTG AGTTCTGTTA CAAGTCAAGG 2520

GCCGAGGCGA AACGAATGAA GGTGGCAAAG AATGCACAGA ATATTAACCC ATCTTCCTCG 2580

CAGAATTCAC AGAATTTTGC AACTTATAAG GAAGGTTACA ACGTATATGG CATCGAAAGT 2640

GTTAAAATT 2649

(6) INFORMATION FOR SEQ ID NO:5:

(i) SEQUENCE CHARACTERISTICS:

(A) LENGTH: 60 base pairs
(B) TYPE: nucleic acid
(C) STRANDEDNESS: both
(D) TOPOLOGY: linear

(ii) MOLECULE TYPE: cDNA

(ix) FEATURE:
(A) NAME/KEY: CDS
(B) LOCATION: 1..60

(xi) SEQUENCE DESCRIPTION: SEQ ID NO:5:

ATG CAG CAC ATT TTT GCC TTC TTC TGC ACC GGT TTC CTA GGC GCG GTA 48
Met Gln His Ile Phe Ala Phe Phe Cys Thr Gly Phe Leu Gly Ala Val
1               5                   10                  15

GTA GGT GCC AAT
60
Val Gly Ala Asn
            20

(7) INFORMATION FOR SEQ ID NO:6:

(i) SEQUENCE CHARACTERISTICS:
(A) LENGTH: 60 base pairs
(B) TYPE: nucleic acid
(C) STRANDEDNESS: both
(D) TOPOLOGY: linear

(ii) MOLECULE TYPE: cDNA

(ix) FEATURE:
(A) NAME/KEY: CDS
(B) LOCATION: 1-60

(xi) SEQUENCE DESCRIPTION: SEQ ID NO:6:
TTT GCT TTG TCG CAA 15
Phe Ala Leu Ser Gln
1               5

CTC ACA GAG CCC CCG AAG CTG CTC CCC CAG ATT GAT ATT GTG AAC 60
Leu Thr Glu Pro Pro Lys Leu Leu Pro Gln Ile Asp Ile Val Asn
                10                  15                  20

(8) INFORMATION FOR SEQ ID NO:7:

(i) SEQUENCE CHARACTERISTICS:
(A) LENGTH: 60 base pairs
(B) TYPE: nucleic acid
(C) STRANDEDNESS: both
(D) TOPOLOGY: linear

(ii) MOLECULE TYPE: cDNA

(ix) FEATURE:
(A) NAME/KEY: CDS
(B) LOCATION: 1-60

(xi) SEQUENCE DESCRIPTION: SEQ ID NO:7:

CAA TCG ATT CCT TGC ATG AGC CAC AGT 27
Gln Ser Ile Pro Cys Met Ser His Ser
1               5

TCA GGG ATG CCC TTG GGA GCC ACG GGA TTG TAA 60
Ser Gly Met Pro Leu Gly Ala Thr Gly Leu
10                  15

(9) INFORMATION FOR SEQ ID NO:8:

(i) SEQUENCE CHARACTERISTICS:
(A) LENGTH: 60 base pairs
(B) TYPE: nucleic acid
(C) STRANDEDNESS: both
(D) TOPOLOGY: linear

(ii) MOLECULE TYPE: cDNA

(xi) SEQUENCE DESCRIPTION: SEQ ID NO:8:

ATGCAAAAGA TTATGCATAT TTCTGTCCTC CTTTCTCCTG TTTTATGGGG ACTGATTTTT 60

(10) INFORMATION FOR SEQ ID NO:9:

(i) SEQUENCE CHARACTERISTICS:
(A) LENGTH: 60 base pairs
(B) TYPE: nucleic acid
(C) STRANDEDNESS: both
(D) TOPOLOGY: linear

(ii) MOLECULE TYPE: cDNA

(xi) SEQUENCE DESCRIPTION: SEQ ID NO:9:

GTAGGGATGG TTCAGTTTTC CACTTCGGAG TTCAGACTGA CACCCCACAT CGACAATTTG 60

(11) INFORMATION FOR SEQ ID NO:10:

(i) SEQUENCE CHARACTERISTICS:
(A) LENGTH: 60 base pairs
(B) TYPE: nucleic acid
(C) STRANDEDNESS: both

(D) TOPOLOGY: linear

(ii) MOLECULE TYPE: cDNA

(xi) SEQUENCE DESCRIPTION: SEQ ID NO:10:

AATTTTGCAA CTTATAAGGA AGGTTACAAC GTATATGGCA TCGAAAGTGT TAAAATTTAA 60